# EUROPEAN PATENT APPLICATION

(11) **EP 1 618 881 A1**
(43) Date of publication of application: **25.01.2006**
(21) Application number: 04017124.1
(22) Date of filing: 20.07.2004
(51) Int. Cl.: A61K 31/56, A61P 21/00

(54) **Use of non-glucocorticoid steroids for the treatment of muscular dystrophy**

(71) Applicant: Santhera Pharmaceuticals (Schweiz) GmbH, 4410 Liestal (CH)
(72) Inventor: Meier, Thomas, Dr., 5065 Basel (CH); Courdier-Fruh, Isabelle, 68330 Huningue (FR); Magyar, Josef P., Dr., 5304 Endingen (CH)
(74) Representative: Grünecker, Kinkeldey, Stockmair & Schwanhäusser Anwaltssozietät

(57) **Abstract**

The invention relates to the therapeutic use of certain classes of steroid compounds for treatment of muscular diseases, in particular muscle diseases caused by mutations in the gene encoding for dystrophin (Duchenne Muscular Dystrophy, DMD, and Becker Muscular Dystrophy, BMD). The steroid compounds increase the levels of the dystrophin-related protein utrophin in cultured human muscle cells derived from donors affected with Duchenne Muscular Dystrophy.

## Description

The present invention relates to the use of non-glucocorticoid steroids for the preparation of a medicament for treating neuromuscular diseases. The invention further relates to pharmaceutical preparations containing a non-glucocorticoid steroid as an active agent.

Duchenne muscular dystrophy (DMD) is a recessively inherited progressive form of muscle-wasting disease occurring world-wide with an incidence of ~1 in 3'000 male births. First signs of the disease become apparent when boys start to walk. Muscle wasting occurs first in proximal and later in distal muscle groups leading to the loss of ambulation in teenage patients. Mutations in the dystrophin gene and absence of dystrophin protein ultimately lead to death of DMD patients at early adulthood, mainly because of respiratory or cardiac failures. Clinical measures to improve quality of life comprise orthopedic surgery and night-time ventilation. Becker muscular dystrophy (BMD) is caused by different mutations of the same dystrophin gene but has a milder clinical course and the patients have a prolonged life expectancy. Cellular processes underlying DMD-associated muscle wasting include the loss of skeletal muscle fibers and accompanying invasion by connective and adipose tissue, clinically observed as pseudo-hypertrophy.

Both DMD and BMD are caused by mutations in the dystrophin gene. The dystrophin gene consists of 2700 kbp and is located on the X chromosome (Xp21.2, gene bank accession number: M18533). The 14 kbp long mRNA transcript is expressed predominantly in skeletal, cardiac and smooth muscle and to a limited extent in the brain. The mature dystrophin protein has a molecular weight of ~427 kDa and belongs to the spectrin superfamily of proteins (Brown S.C., Lucy J.A. (eds), "Dystrophin", Cambridge University Press, 1997). While the underlying mutation in DMD leads to a lack of dystrophin protein, the milder BMD-phenotype is a consequence of mutations leading to the expression of abnormal, often truncated, forms of the protein with residual functionality.

The N-terminal part of dystrophin binds to actin filaments of the cytoskeleton, whereas domains in the C-terminal part of the dystrophin molecule bind to the membrane associated β-dystroglycan. Therefore, dystrophin serves as a molecular linker between the cytoskeleton and the muscle cell membrane and, indirectly, via the so-called dystrophin-associated protein complex (DAPC) also to the extracellular matrix. Known binding partners of dystrophin also include syntrophin, dystrobrevin, the neuronal type nitric oxide synthase (nNOS) and the sarcoglycan-sarcospan (SS) complex. These protein interactions involving both the carboxy- and aminoterminal region of the dystrophin protein are thought to contribute to the mechanical stability of the muscle cell membrane during cycles of contraction and relaxation. Dystrophin is also important for the assembly or integrity of the DAPC-complex itself, as it has been shown that in dystrophin-deficient muscle cells of DMD patients many components of the DAPC complex are reduced or absent in the sarcolemma.

Within the spectrin superfamily of proteins, dystrophin is closest related to utrophin (gene bank accession number: X69086), to dystrophin related protein-2 (gene bank accession number: NM001939) and to dystrobrevin (gene bank accession number: dystrobrevin alpha: BC005300, dystrobrevin beta: BT009805). Utrophin is encoded on chromosome 6 and the ~395 kDa utrophin protein is ubiquitously expressed in a variety of tissues including muscle cells. The N-terminal part of utrophin protein is 80% identical to that of dystrophin protein and binds to actin with similar affinity. Moreover, the C-terminal region of utrophin also binds to β-dystroglycan, α-dystrobrevin and syntrophins. Utrophin is expressed throughout the muscle cell surface during embryonic development and is replaced by dystrophin during postembryonic development. In adult muscle utrophin protein is confined to the neuromuscular junction. Thus, in addition to sequence and structural similarities between dystrophin and utrophin, both proteins share certain cellular functions. Consequently, it has been proposed that upregulation of utrophin could ameliorate the progressive muscle loss in DMD and BMD patients and offers a treatment option for this devastating disease (WO96/34101).

Experimental evidence that supports this hypothesis stems from results obtained with the mdx-mouse, a generally accepted animal model for DMD (Allamand & Campbell, 2000, *Hum Mol. Genetics* 9: 2459). The mdx-mouse carries a pre-mature stop codon in the dystrophin gene and, like DMD patients, lacks functional dystrophin protein. Overexpression of utrophin gene constructs in the mdx-mouse using transgenic methods or viral vectors results in normalization of histological and physiological parameters normally associated with dystrophin deficiency (Tinsley, J. M., Potter, A. C., et al., 1996, *Nature* 384:349-53. Yang, L., Lochmuller, H., et al., 1998, *Gene Ther*.;5:369-79. Gilbert, R., Nalbantoglu, J., et al., 1999, *Hum Gene Ther,* 10:1299-31). Moreover, high levels of utrophin expression in non-muscle tissues have no toxic effect (Fisher R., Tinsely J.M., 2001, *Neuromuscul Disord* 11: 713-21). These findings in a widely accepted and disease-relevant animal model represent a proof of concept for the treatment of DMD/BMD by elevation of utrophin protein levels.

Besides gene therapeutic approaches involving utrophin gene transfer increased expression of utrophin protein can also be achieved by transcriptional activation of one of the two known promoters for utrophin, termed "utrophin promoter A" and "utrophin promoter B" respectively (promoter A and B Gene Bank Accession Number: utrophin promoter A: X95523; utrophin promoter B: AJ250044, W096/341 01 A, WO01/25461A1). In myotubes, transcription of utrophin is predominantly initiated from promoter A, which contains a binding site (termed N-box) for the GA-binding protein (GABPα/β) (Gramolini A.O., Angus L.M., 1999, *Proc. Natl. Acad. Sci USA* 96: 3223-3227; Khurana T.S., Rosmarin A.G. et al., 1999, *Mol Biol Cell* 10: 2075-86), a binding site (E-box) for myogenic regulatory factors (Perkins K.J., Burton E.A. et al., 2001, *Nucleic Acid Res* 29: 4843-50) and binding sites for the more ubiquitous transcription factors Sp1 and Ap-2 as well as for the nuclear factor of activated T cells (NFAT). The latter is activated by the calcium-responsive intracellular protein phosphatase calcineurin. Transgenic overexpression of calcineurin and activation of NFAT has been shown to ameliorate the dystrophic phenotype in mdx-mice (Chakkalakal J.V., Harrison M.A., 2004, *Hum Mol Genet* 13: 379-88). Alternatively, utrophin protein content could be increased by improving the stability of the utrophin protein.

Using pharmacological methods, increase of utrophin protein content in human muscle cells has been reported for glucocorticoid-steroids. Such glucocorticoids include for example α-methylprednisolone, dexametasone, triamcinolone acetonide, halcinonide, dichlorisone, and fluocinolone acetonide (Courdier-Fruh, I., Barman, L., Briguet, A. & Meier, T, 2002, *Neuromuscul. Disord.,* 12, Suppl:S95).

However, glucocorticoids cause severe clinical side effects such as weight gain, diabetes mellitus, peptic ulcer, Cushing's syndrome, osteoporosis, skin atrophy, psychosis, glaucoma and many others which prevent the long term application of this class of chemical compounds. There is currently a high need to develop non-glucocorticoid based pharmacological therapies for the treatment of DMD and BMD.

An object underlying the present invention is the provision of means and modes for treating diseases responsive to the increase of utrophin levels, in particular of utrophin levels in human muscle cells.

Said object is achieved by a pharmaceutical preparation comprising as active agent a compound selected from steroids having no glucocorticoid-like pharmacological activity (termed "non-glucocorticoid steroids" thereafter).

Said object is further achieved by using a compound or compound combination as further defined herein for the preparation of a medicament for the treatment of a disease responsive to the increase of utrophin protein levels and/or associated with loss of the dystrophin-DAPC complex.

Surprisingly it was found that steroid compounds having pharmacological properties different from glucocorticoids also increase the level of utrophin protein, particularly in muscle cells derived from human DMD donors, as detected by a cell-based ELISA technique. Therefore, these non-glucocorticoid steroids represent a novel pharmacological means for the treatment of diseases susceptible to the increase of utrophin protein levels.

The best-characterized cellular response of glucocorticoids is the regulation of gene transcription. Distinct protein domains characterize nuclear receptors including glucocorticoid receptors (GRs). Interaction of the glucocorticoid with the ligand binding domain (LBD) liberates the glucocorticoid receptor from interacting chaperones and ensures selectivity of the physiological response. Upon activation and translocation to the nucleus, glucocorticoid receptor homodimers are recruited via their DNA-binding domains to specific DNA sequences, the so called glucocorticoid response elements (GREs), which typically are inverted hexanucleotide repeat sequences separated by one to several base pairs (Beato M, et al., 1995, *Cell:* 83: 851-857; Iniguez-Lluhi J.A. et al., 1997, *J. Biol. Chem.* 272: 4149-56). Upon DNA-binding the glucocorticoid receptor interacts with the transcription apparatus and in conjunction with specific transcription factors and co-activators regulates gene expression (Freedman LP, 1999, Cell: 97:5-8). This mechanism of action, called transactivation, is thought to be responsible for several severe side effects associated with glucocorticoids. It is assumed that binding of compounds to the glucocorticoid receptor triggers this transactivation pathway which results in the severe side effects of glucocorticoids. (Schäcke H. et al., *Proc. Natl. Acad. Sci. USA* 101:227-232). A method to identify glucocorticoid-like activity of chemical compounds using promoter-reporter constructs is described in Courdier-Fruh I. et al., 2003, *Neuromuscular Disord.* 13:699-704. The non-glucocorticoid compounds for use in accordance with the present invention, hence, do not bind to glucocorticoid receptors. Alternatively, non-glucocorticoid steroids may be characterized by not being inhibited by glucocorticoid receptor antagonists, such as RU38486.

Here we describe the surprising finding that non-glucocorticoid steroids offer novel therapeutic approaches to treat DMD and BMD and related forms of muscle wasting associated with dystrophin deficiencies. This is based on the result that several classes of steroids, including bile acids (i.e. cholanic acids), bis-nor- and etiocholanic acids, spirostanes, sterols, androstanes, estranes, pregnanes, estratrienes and cardenolides increase the level of utrophin protein in human muscle cells derived from DMD patients comparable to the level of utrophin protein obtained in muscle cells treated with the glucocorticoid 6α-methylprednisolone-21 sodium succinate (PDN). The generally well-accepted absence of glucocorticoid-specific clinical side-effects from the non-glucocorticoid steroids disclosed herein make these compounds suitable means for the treatment of "dystrophinopathies", such as DMD and BMD and related muscle diseases that are associated with a loss of dystrophin or the dystrophin-associated glycoprotein complex.

The method used to detect and determine utrophin-inducing activity of non-glucocorticoid steroids has been described previously (Courdier-Fruh, I., Barman, L., Briguet, A. & Meier, T.; 2002, *Neuromuscul. Disord.;* 12 Suppl:S95). Briefly, primary human muscle cell cultures were prepared according to standard protocols (Askanas V, Kwan H, Alvarez RB, Engel WK, Kobayashi T, Martinuzzi A, Hawkins EF., 1987, *J Neurocytol.,* 16:523-537). Test compounds and the glucocorticoid 6α-methylprednisolone-21 sodium succinate (Pharmacia & Upjohn; Solu-Medrol®, PDN) as reference compound were applied at 500 nM final concentration as soon as myoblasts started to differentiate into myotubes. Incubation with the test compound was ended as soon as well differentiated myotubes have formed, typically resulting in an incubation time with a test compound for 3-7 days.

Normalized concentrations of utrophin protein in human muscle cells cultured in microtiter plates were determined by a cell-based enzyme-linked immunosorbent assay (ELISA) procedure that allows a successive readout for the cell density and utrophin protein level. For calibration, the cell density and differentiation was determined by absorbance measurements of the total dehydrogenase enzyme activity in each well using the colorimetric CellTiter 96®AQ One Solution Reagent Proliferation Assay (Promega) according to the manufacturer's recommendation. Subsequently, cells were fixed, washed, permeabilized with 0.5% (v/v) Triton X-100 and unspecific antibody binding-sites were blocked by standard procedures. Utrophin protein levels were determined immunologically with utrophin-specific primary antibodies (mouse monoclonal antibody to the amino terminal portion of utrophin, NCL-DRP2, Novocastra Laboratories) and with appropriate peroxidase-coupled secondary antibodies using QuantaBluTM Fluorogenic Peroxidase Substrate Kit (Pierce) for detection. Fluorescence measurements were carried out with a multilabel counter (Wallac) at λₑₓ = 325nm and at λₑₘ = 420nm. The primary readout of this signal is presented in arbitrary units. For calibration, the arbitrary units representing the relative utrophin protein content of each well were divided by the corresponding cell-titer absorbance value to correct for cell density. For comparison between experiments, the cell-titer corrected readout for utrophin protein content in each well was expressed in per cent of solvent treated control cultures (set to 100%). PDN was included in all experiments as reference glucocorticoid, N=3-5 for each compound tested.

Non-glucocorticoid test compounds, applied in a concentration of 500 nM, were regarded as "positive" in case the increase in the level of utrophin protein was at least 20% (mean value) over control cultures treated with the appropriate dilution of DMSO solvent as control and containing no test compound.

This category of non-glucocorticoid steroids that induces utrophin protein content in muscle cells can be represented by the following general description and formula.

The compounds have the tetracyclic backbone common to all steroids with one or two angular methyl groups in position 10 and/or 13 and an oxygen function, e.g. a hydroxy- or acyloxy group (deduced from lower aliphatic, cycloaliphatic, aromatic or heteroaromatic carboxylic acids), a carbonyl group or a halogen atom in position 3. Further, the usual saturated or unsaturated 1-, 2-, 3-, 5- 8-, 9-, or 10-carbon atom side chain in position 17 and carrying optionally oxygen functions at various positions on the steroid backbone, preferably at least at one position selected from any of positions 5, 6, 7, 9, 11, 12, 14, 16, 17, 18, 19, 20 and 21 may be present. In addition, saturated and non-saturated lower alkyl groups, (preferably methyl-, ethyl-, propyl-, butyl; C₁-C₄) in at least one of positions 4, 6, 7, 14, 17 or 24 may be present. Moreover, at least one double bond, preferably in 1-, 3-, 4-, 5(6)-, 5(10)-,6-, 7-, 8(9)-, 8(14)-, 14-, 16-, 22- or 25-position may be present. Finally, at least one saturated or unsaturated carbocyclic, heterocyclic, aromatic or heteroaromatic substituents attached to C(17) may be present.

The following general formula (I) represents non-glucocorticoid steroids that increase utrophin protein levels in human muscle cells:

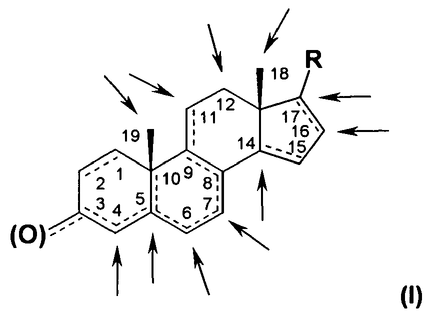

Arrows in the general formula indicate possible points of attachment of substituents; R: alternative side chains at C(17).

Examples of back bones of alternative side chains at C(17): (R)

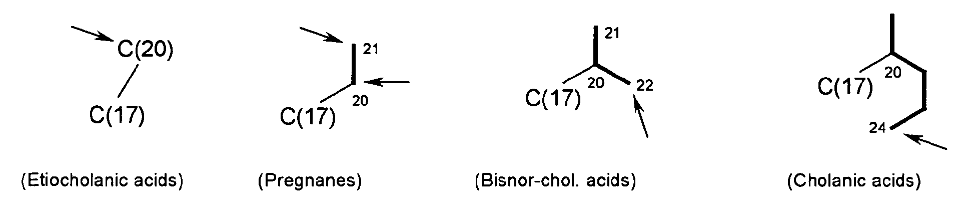

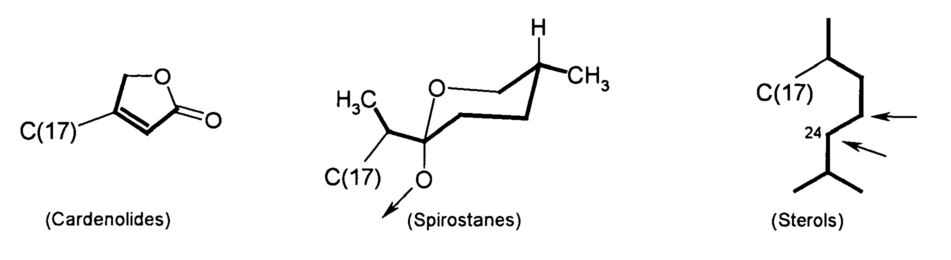

Arrows in the list of alternative side chains at C(17) indicate possible points of attachment of substituents.

The following classes of non-glucocorticoid steroids were surprisingly found to increase levels of utrophin protein in muscle cells derived from DMD patients and, consequently, offer novel treatment means in particular for DMD, BMD and related muscle disorders:
1. Bile acids, more preferred cholanic acids and their esters, contain an oxygen function in position 3 and optionally in position 6, 7, 11 and 12.
   A preferred group is shown by the following formula. 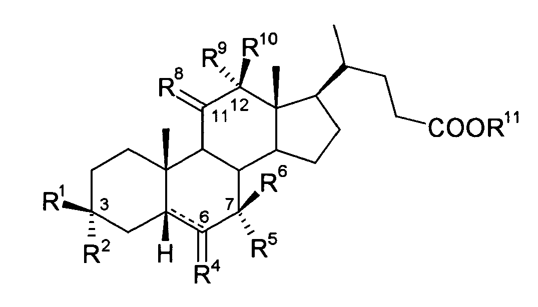
   The residue in position 17 defines this steroid class as a cholanic acid (or ester). The remaining residues besides the oxygen function in position 3 are optional for this class of steroid compounds.
2. Bis-nor- and etiocholanic acids described as steroidal acids and esters contain an oxygen function at C(3) and a carboxylic group or a 2-propionic acid residue in position 17 and optionally additional oxygen functions in 7-, 11-, 12-, 14-, and 16-positions and/or a Δ⁵ or Δ⁶ double bond.
   A preferred group is shown by the following formulae: 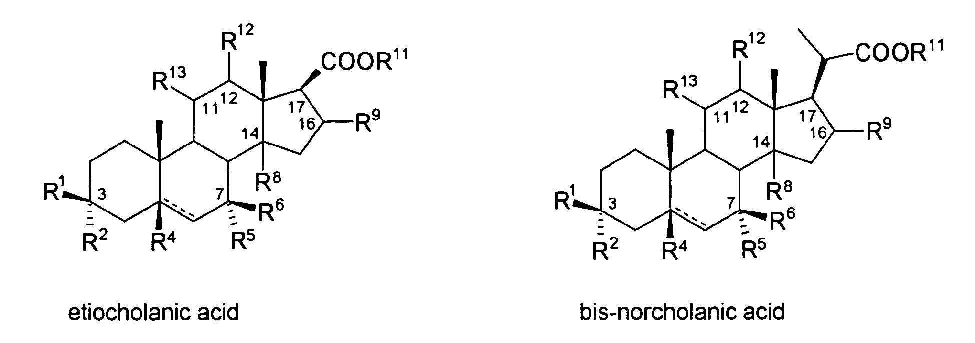
3. Spirostanes contain an oxygen function in position 3 and the structural element as shown below at position 16 and 17, the substituents thereof being not mandatory. These compounds optionally comprise a Δ⁹⁽¹¹⁾ or Δ¹¹⁽¹²⁾ double bond and an oxygen function in 11 and/or 12 position.
   A preferred group is shown by the following formula. 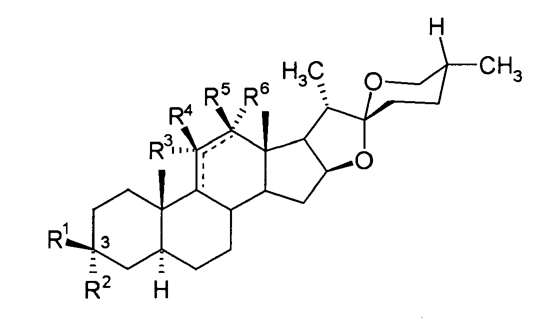
4. Sterols with a cholestane backbone contain an oxygen function in position 3 and a side chain in position 17. Optionally, they contain additional oxygen functions in various positions of the cholestane backbone, preferably at position 4, 5, 6, and 7 and/or alkyl groups in position 4, 14 and/or in the side chain attached to position 17.
   A preferred group is shown by the following formula: 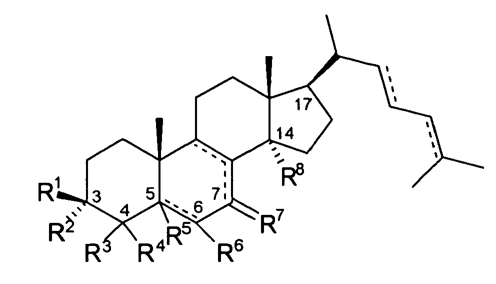
5. Androstanes and estranes, more preferred steroids having an androstane, 19-norandrostane (estrane) or 18-homoestrane (18-methyl-estrane) skeleton, contain at C(3) an oxygen function or a halogen substituent, preferably a chlorine, fluorine, or bromine atom and optionally an additional oxygen function in position 16 or 17, optionally a 17α-alkyl, - alkenyl or alkynyl group or optionally a Δ⁴-, Δ⁵- or a Δ¹⁶-double bond.
   A preferred group is represented by the following formula: 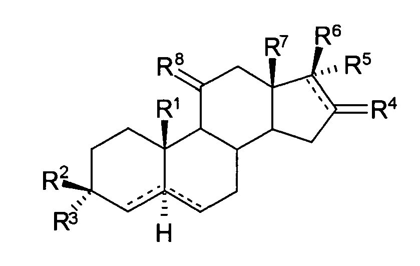
6. Pregnanes (excluding glucocorticoids) have an oxygen function each in position 3 and 20 and optionally at least one additional oxygen function in position 4, 5, 9, 11, 12, and/or 21 as at least one double bond, for instance Δ¹-, Δ⁴-, Δ⁵-, Δ⁹⁽¹¹⁾ - and/or a Δ¹⁶⁻double bond.
   A preferred group is shown by the following formula: 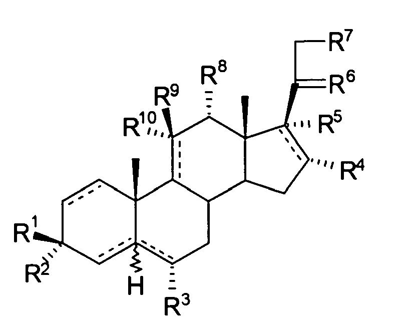
7. Estratrienes, more preferred 19-norsteriods, containing the structural features of estrogens (aromatic ring A), contain oxygen functions in position 3 and 17. Optionally, they contain an oxygen function at C(16) and/or a saturated alkyl group in position 17α, as well as a further optional additional double bond between C(6) and C(7).
   A preferred group is represented by the following formula: 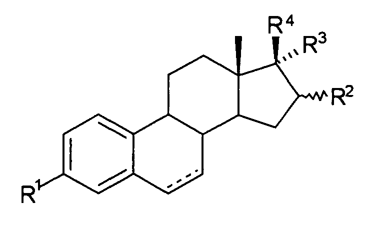
8. Cardenolides are characterized by the tetracyclic steroid backbone with an α,β-unsaturated lactone ring attached to C(17) and an oxygen function in position 3. Optionally, they further contain at least an additional oxygen function, preferably in positions 5, 11, 12, 14, and/or 19. Moreover, they may contain further double bonds, preferably between C(14) and C(15).
   A preferred group is shown by the following formula:

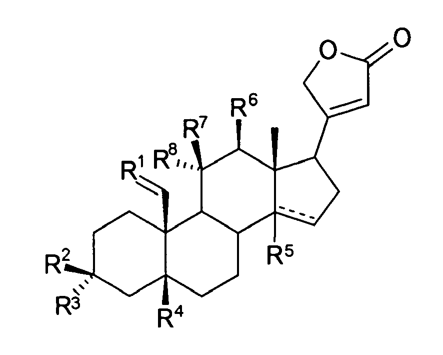

1. Preferred substituents of bile acids comprise:

| | | |
|---|---|---|
| R¹ + R² = O | R⁵ + R⁶ = O | R⁵ = OR⁷; R⁶ = H |
| R¹= OR³; R²=H | R⁵ + R⁶ = H₂ | R⁸ = O; H₂ |
| R¹ = H; R² = OR³ | R⁵=H; R⁶ = OR⁷ | R⁹ + R¹⁰ = O; H₂ |
| R³ = H; acyl* | R⁷ = H; acyl* | R⁹ = OR⁷; R¹⁰ = H |
| R⁴ = H₂; O | | R¹¹ = H; Alkyl** |

| | | |
|---|---|---|
| *) corresponding to lower (C₁-C₄) aliphatic, cycloaliphatic, aromatic or heteroaromatic carboxylic acids | | |
| **) lower saturated and unsaturated (C₁-C₁₀) alkyl group, or a benzylic, aromatic and heteroaromatic residue | | |

2. Preferred substituents of bis-nor- and etiocholanic acids comprise:

| | | |
|---|---|---|
| R¹ + R² = O | R⁵ + R⁶ = O | R⁵ = OR⁷; R⁶ = H |
| R¹ = OR³; R² = H | R⁵ + R⁶ = H₂ | R⁸ = α- or β-H; β-OH |
| R¹ = H; R² = OR³ | R⁵ = H; R⁶ = OR⁷ | R⁹ = OR⁷ |
| R³ = H; acyl* | R⁷ = H; acyl* | |
| R⁴ = α- or β-H | | |
| | R¹¹ = H; Alkyl** | |
| | R¹² = H; OR⁷ | |
| | R¹³ = H, OR⁷ | |

| | | |
|---|---|---|
| *) corresponding to lower (C₁-C₄) aliphatic, cycloaliphatic, aromatic or heteroaromatic carboxylic acids | | |
| **) lower saturated and unsaturated (C₁-C₁₀) alkyl group, or a benzylic, aromatic and heteroaromatic residue | | |

3. Preferred substituents of spirostanes comprise:

| | | |
|---|---|---|
| R¹ = OR⁷ ; R² = H | R³ = OR⁷; R⁴ = H | R⁵ = OR⁷; R⁶ = H |
| R¹ = H; R² = OR⁷ | R³= H; R⁴ = OR⁷ | R⁵ = H; R⁶ = OR⁷ |
| R¹ + R² = O | R³ + R⁴ = O, H₂ | R⁵ + R⁶ = O, H₂ |
| | | |
| | R⁷ = H, Acyl* | |

| | | |
|---|---|---|
| *) corresponding to lower (C₁-C₁₀) aliphatic, cycloaliphatic, aromatic or heteroaromatic carboxylic acids | | |

4. Preferred substituents of sterols comprise:

| | | |
|---|---|---|
| R¹ =OR⁹; R²=H | R³ = R⁴ = H; CH₃ | R⁷ = O; H₂ |
| R¹ = H; R² = OR⁹ | R⁵=H; OH | R⁸ = H; CH₃ |
| R¹ + R² = O | R⁶ = H; OR⁹ | R9 = H; Acyl* |

| | | |
|---|---|---|
| *) corresponding to lower (C₁-C₆) aliphatic, cycloaliphatic, aromatic or heteroaromatic carboxylic acids | | |

5. Preferred substituents of androstanes / estranes comprise:

| | | |
|---|---|---|
| R¹ = H; CH₃ | R⁴ = H₂; O | R⁵ + R⁶ = O; H₂ |
| R² = OH; OAcyl*; Hal | R⁵ = H; alkyl; alkenyl; alkynyl | R⁷ = CH₃; C₂H₅ |
| R³ = H | | R⁸ = H₂; O |
| R² + R³ = O | R⁶ = H; OH; OAcyl* | |

| | | |
|---|---|---|
| *) corresponding to lower (C₁-C₆) aliphatic, cycloaliphatic, aromatic or heteroaromatic carboxylic acids | | |

6. Preferred substituents of pregnanes comprise:

| | | |
|---|---|---|
| R¹ = OH; OAcyl* | R⁴ = R⁵ = H | R⁸ = H; OH; OAcyl* |
| R² = H | R⁴ + R⁶ = epoxy | R⁹ = H; OH |
| R¹ + R² = O | R⁶ = -O-CH₂-CH₂-O-; O | R¹⁰ = H; OH; OAcyl* |
| R³ = H; CH₃ | R⁷ = H; OH; OAcyl* | |

| | | |
|---|---|---|
| *) corresponding to lower (C₁-C₆) aliphatic, cycloaliphatic, aromatic or heteroaromatic carboxylic acids | | |

7. Preferred substituents of estratrienes comprise:

| |
|---|
| R¹ = OH, OAcyl* |
| R² = OH; OAcyl*; H |
| R³ = H; OH; OAcyl*; alkyl** |
| R⁴ = H; OH; OAcyl* |
| R³ + R⁴ = O |

| |
|---|
| *) corresponding to lower (C₁-C₄) aliphatic, cycloaliphatic, aromatic or heteroaromatic carboxylic acids |
| **) lower saturated and unsaturated (C₁-C₁₀) alkyl group, or benzylic, aromatic and heteroaromatic residues |

8. Preferred substituents of cardenolids comprise:

| | | |
|---|---|---|
| R¹ = H₂; O | R⁴ = H; OH | R⁷ = H; OH, OAcyl* |
| R² = H; OR⁹ | R⁵ = H; OH | R⁸ = H; OH; OAcyl* |
| R³ = H; OR⁹ | R⁶ = H; OH; OAcyl* | R⁷ + R⁸ = O |
| R² + R³ = O | | R⁹ = H; Acyl* |

| | | |
|---|---|---|
| *) corresponding to lower (C₁-C₆) aliphatic, cycloaliphatic, aromatic or heteroaromatic carboxylic acids | | |

The compound which is suitable as the active agent in the pharmaceutical agent for use according to the invention is capable of inducing elevated levels of utrophin protein when brought into contact with the respective cells, preferably human muscle cells, more preferably human muscle cells deficient in dystrophin. The assay for measuring whether a compound induces elevated levels of utrophin protein has been described above. The effect of the compound may be exerted at the transcriptional or translational level or by reducing the turnover of utrophin protein or by any means that increases the stability of utrophin mRNA or protein. The final outcome is that the cell having been in contact with the compound shows an increased level of utrophin protein.

In a further preferred embodiment the compound is selected from bile acid analogs, bis-nor- and etiocholanic acids, spirostanes, sterols, androstanes, estranes, pregnanes, estratrienes, and cardenolides. The structural elements being characteristic for each of these classes are shown above.

Surprisingly, it has been found that practically each class of non-glucocorticoid steroids is capable of increasing the utrophin protein level in a cell. By using the screening assay as described herein, it is well within the routine of the skilled person to identify within the non-glucocorticoid steroids those compounds that are capable of increasing the utrophin protein level to a degree that renders the compound suitable in the pharmaceutical preparation for use according to the present invention.

Preferably, the compound for use in the pharmaceutical preparation is capable of increasing the level of utrophin in muscle cells, preferably in human muscle cells, most preferably in human muscle cells derived from BMD or DMD patients.

In a further preferred embodiment, the compound for use according to the invention is capable of increasing the level of utrophin protein by at least 10%, preferably at least 20%, more preferably at least 50%, most preferably 100% over the level observed when incubating the cell with a solvent only and compound-free control. In said assay of examining a compound's capability to increase the utrophin level, the compound in question is applied in a concentration of about 500 nM.

In a further preferred embodiment, the non-glucocorticoid steroid compound is combined with a further active agent, wherein the steroid compound and the further active agent can be used simultaneously, separately or sequentially in order to treat the disease in question. The two active agents may be provided in a single dosage form or as separate formulations, each formulation containing one of the two active agents.

In a further preferred embodiment, the further active agent is suitable for treating Duchenne Muscular Dystrophy or Becker Muscular Dystrophy.

Preferably, such an agent is selected from an antioxidant, creatine and glucocorticoids. Suitable antioxidants are Vitamin E, CoQ10 and idebenone.

In a further preferred embodiment, the further active agent is selected from inhibitors for calcium dependent proteases (calpains), more preferably inhibitors as disclosed in PCT/EP2004/002142.

In a further preferred embodiment, the further active agent is selected from inhibitors of the 20S proteasome, preferably bortezomib (Velcade®).

The diseases to be treated with the pharmaceutical preparation according to the invention are diseases susceptible to an increase of dystrophin or utrophin levels or diseases that are associated with the loss of the dystrophin DAPC-complex.

Patients having said disease show a reduced expression and/or protein level of dystrophin and/or members of the DAPC-complex. In such patients the protein level of either dystrophin, dystroglycans or sarcogylcans is reduced, in particular, in muscle cells when compared to the protein level in muscle cells of healthy symptom-free patients.

In a further preferred embodiment the disease is characterized by a loss of dystrophin which loss may lead to a 100% loss of dystrophin protein or to substantial reduction of dystrophin protein, in particular in muscle cells when compared with healthy patients.

In a further preferred embodiment the diseases are neuromuscular diseases, such as muscular dystrophies, including dystrophinopathies and sarcoglycanopathies, limb girdle muscular dystrophies, congenital muscular dystrophies, congenital myopathies, distal and other myopathies and myotonic syndromes.

In a further preferred embodiment the disease is a muscular dystrophy or a related muscle wasting disorder associated with dystrophin deficiency.

In a further preferred embodiment the disease to be treated is Duchenne Muscular Dystrophy or Becker Muscular Dystrophy.

Preferred modes of administration are oral, i.p., i.v., i.m., s.c., parenteral, intranasal and transdermal, whereas oral is the most preferred mode of administration.

### Administration and Dose Ranges

Any suitable route of administration may be employed for providing a mammal, especially a human, with an effective dosage of the non-glucocorticoid steroids as described here. For example, oral, rectal, topical, parenteral, ocular, pulmonary or nasal administration may be employed. Dosage forms include, for example, tablets, troches, dispersions, suspensions, solutions, capsules, creams, ointments and aerosols.

The effective dosage of the active ingredient employed may vary depending on the particular compound employed, the mode of administration, the condition being treated and the severity of the condition being treated. Such dosage may be ascertained readily by a person skilled in the art.

When treating Duchenne Muscular Dystrophy, Becker Muscular Dystrophy and other muscular dystrophies, generally, satisfactory results are obtained when the compounds of the present invention are administered at a daily dosage of about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

### Formulation

The non-glucocorticoid steroids disclosed herein are preferably formulated into a dosage form prior to administration. Accordingly, the present invention also includes a pharmaceutical composition comprising a non-glucocorticoid steroid and a suitable pharmaceutical carrier.

The present pharmaceutical compositions are prepared by known procedures using well-known and readily available ingredients. In making the formulations of the present invention, the active ingredient (non-glucocorticoid steroid) is usually mixed with a carrier, or diluted by a carrier, or enclosed within a carrier, which may be in the form of a capsule, sachet, paper or other container. When the carrier serves as a diluent, it may be a solid, semisolid or liquid material, which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents and/or flavoring agents. The compositions of the invention may be formulated so as to provide quick, sustained or delayed release of the active ingredient after administration to the patient

The non-glucocorticoid steroids can be converted in a manner known per se into their salts with physiologically compatible acids, for example hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid. The salt formation is preferably carried out in a solvent, for example diethyl ether, diisopropyl ether, alkyl acetates, acetone and/or 2-butanone. Moreover, trimethylchlorosilane in aqueous solution is suitable for preparing the hydrochlorides.

The substances corresponding to formula I are toxicologically safe, which means that they can be used as a pharmaceutical active agent in medicinal drugs.

The non-glucocorticoid steroids can be combined with excipients, fillers, solvents, diluents, dyes and/or binders. The choice of auxiliary substances as well as the amounts thereof to be used depends on whether the medicinal drug is to be administered orally, intravenously, intraperitoneally, intradermally, intramuscularly, intranasally, buccally or topically. For oral application suitable preparations are in the form of tablets, sugar-coated pills, capsules, granular powders, drops, juices and syrups, while for parenteral, topical and inhalative application suitable forms are solutions, suspensions, easily reconstitutable dry preparations as well as sprays. The steroids can be administered in a sustained-release substance, in dissolved form or in a plaster, optionally with the addition of agents promoting penetration of the skin, and are suitable as percutaneous application preparations. Forms of preparations that can be used orally or percutaneously may produce a delayed release of the compounds.

The following examples illustrate the invention.

### Examples:

### Example 1: Increase of the utrophin protein level by chenodeoxycholic acid (CDCA)

A range of different pharmaceutical compounds were tested for their ability to increase levels of utrophin protein in primary human myotube cultures. The cell-based ELISA protocol used to detect the level of utrophin protein is provided above and described in Courdier-Fruh, I., Barman, L., Briguet, A. & Meier, T, 2002, *Neuromuscul. Disord.:* 12, Suppl:S95. Briefly, fixed and permeabilized human muscle cells derived from DMD patients and cultured in 96-well cell culture plates were incubated with antibodies specific for utrophin at a dilution of 1:1'000 in blocking solution (phosphate buffered saline (PBS) supplemented with 2% fetal calf serum and 1% bovine serum albumin) for 10-15 hours at 4°C. Following several washing steps with 0.1% Triton X-100 in PBS, peroxidase-coupled secondary antibodies diluted 1:3'000 in blocking solution were applied in each well for 1-2 hours at room temperature. Signal detection, data acquisition and normalization was performed as described above and in Courdier-Fruh, I., Barman, L., Briguet, A. & Meier, T, 2002, *Neuromuscul. Disord.:* 12, Suppl:S95.

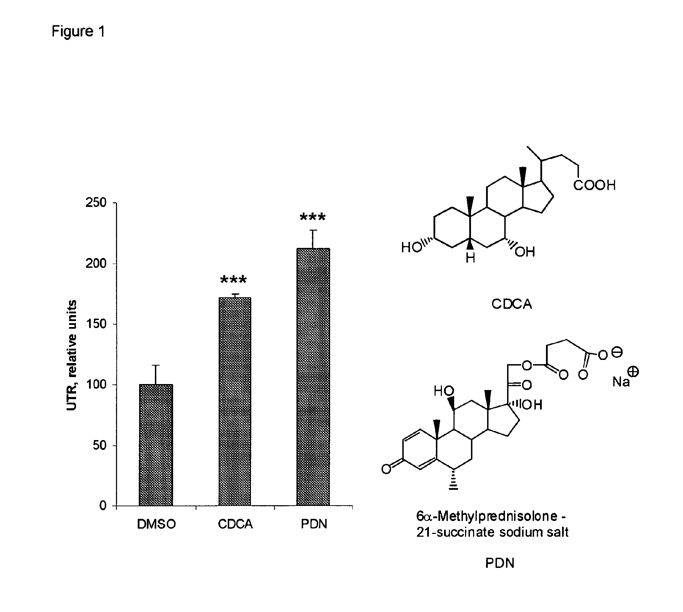

We found that glucocorticoid steroids (such as α-methylprednisolone) elevated levels of utrophin protein in human muscle cells as has been described previously (Courdier-Fruh, I., Barman, L., Briguet, A. & Meier, T, 2002, *Neuromuscul. Disord.:* 12, Suppl:S95). Surprisingly, however, we also found that a steroid compound, 3α,7α-dihydroxy-5β-cholanic acid (chenodiol, chenodeoxycholic acid, CDCA) that is structurally and pharmacologically distinct from glucocorticoids, also elevated levels of utrophin protein in human muscle cells derived from DMD patients (see Fig. 1).
*Figure 1 legend:* Compared to solvent-treated control cultures (DMSO) utrophin protein levels normalized to the cell density per well (UTR, relative units) are elevated up to 200% in human DMD-patient derived muscle cells incubated with 6α-methylprednisolone-21 sodium succinate (PDN). Surprisingly, treatment with 500 nM chenodeoxycholic acid (CDCA) for the same period of time also significantly increased utrophin protein levels. Data is mean ± standard deviation, N= 5; *** p<0.0001 (unpaired t-test).

### Example 2: Induction of utrophin protein in human muscle cells by CDCA is not inhibited by the glucocorticoid receptor antagonist RU38486

An important aspect of the potential therapeutic applicability of utrophin-inducing steroids is the question whether these compounds would induce side effects normally associated with glucocorticoids. These clinical side effects include weight gain, diabetes mellitus, peptic ulcer, Cushing's syndrome, osteoporosis, skin atrophy, psychosis, glaucoma and many others. Consequently, in order to ensure that the bile acid CDCA not only is structurally different from glucocorticoids (such as PDN) but also exert distinct pharmacological properties we tested whether CDCA-mediated utrophin induction would be inhibited by RU38486 (mifepristone), a well-established glucocorticoid antagonist (Agarwali MK, 1996, *Pharmacol Ther* 70: 183-213). For this, human DMD-patient derived muscle cells were treated with 500 nM CDCA in the presence and absence of 500 nM RU38468. In previous experiments it has been documented that PDN-mediated increase in utrophin protein could be inhibited by simultaneous exposure to RU38468 (Fig. 3 in: Courdier-Fruh, I., Barman, L., Briguet, A. & Meier, T, 2002, *Neuromuscul. Disord.,* 12, Suppl:S95). Surprisingly, we now found that the utrophin protein-inducing effect of the bile acid CDCA can not be inhibited by RU38486. Specifically, the level of utrophin protein levels induced in DMD muscle cells was not different when equimolar concentrations of RU38468 were applied to the CDCA-stimulus. Therefore, since RU38468 pharmacologically distinguished CDCA from a typical glucocorticoid such as PDN it is concluded that CDCA and other non-glucocorticoid steroids will not cause the therapy-limiting side effects that are commonly seen with PDN or other glucocorticoids. In summary, CDCA can induce therapeutically relevant levels of utrophin protein but at the same time does not induce the undesirable clinical side effects of glucocoricoid steroids. Therefore, non glucocorticoid steroids that increase utrophin protein levels in DMD patients offer significant clinical benefits.

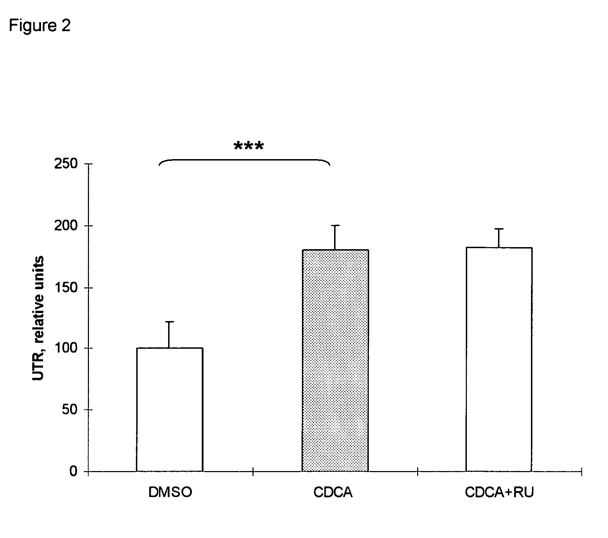

*Figure 2 legend:* Normalized utrophin protein level (UTR, relative units) of human DMD-patient derived muscle cells was increased by treatment with 500 nM CDCA compared to muscle cells treated with appropriate DMSO-solvent controls (DMSO). This increase in utrophin protein levels was not inhibited by simultaneous incubation with 500 nM of the glucocorticoids receptor antagonist RU38486 (CDCA +RU). This result is in clear contrast to previously published data that demonstrate that glucocorticoid-mediated increase in utrophin protein in human muscle cells is suppressed by simultaneous incubation with RU38486. Data is mean ± standard deviation, N= 5-6; *** p<0.0001 (unpaired t-test).

### Examples 3 - 85:

In the following examples, values of normalized utrophin protein levels have been determined as described in examples 1 and 2. The first set of data provided indicate the % increase of normalized utrophin protein over control (DMSO-solvent treated) in human muscle cultures upon incubation with 500 nM of the non-glucocorticoid steroid (data is mean value; N=3-6). The second set of data reflects the normalized level of utrophin protein expressed as % of the PDN-treated sister cultures in the same experiment (data is mean value; N=3-6)

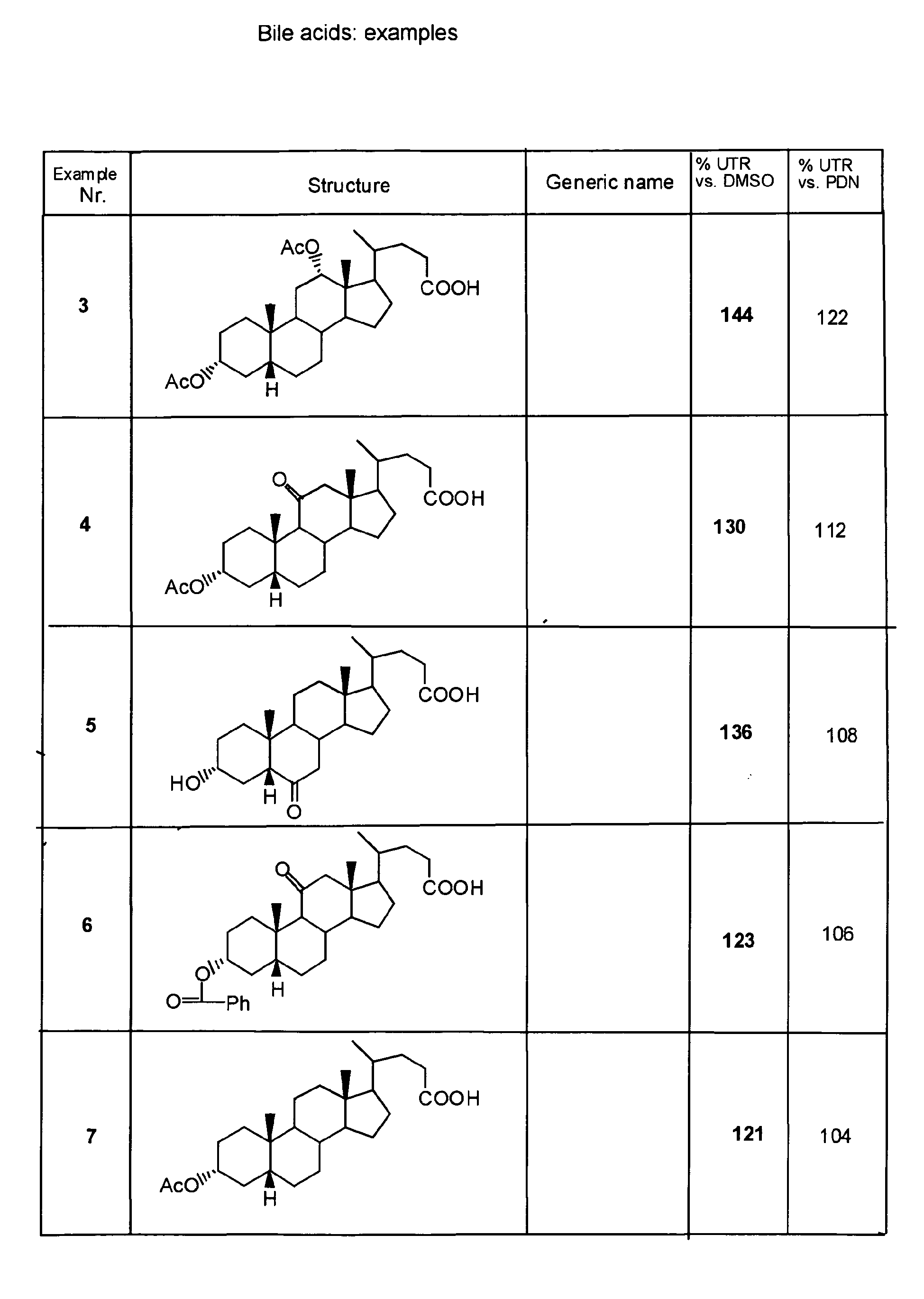

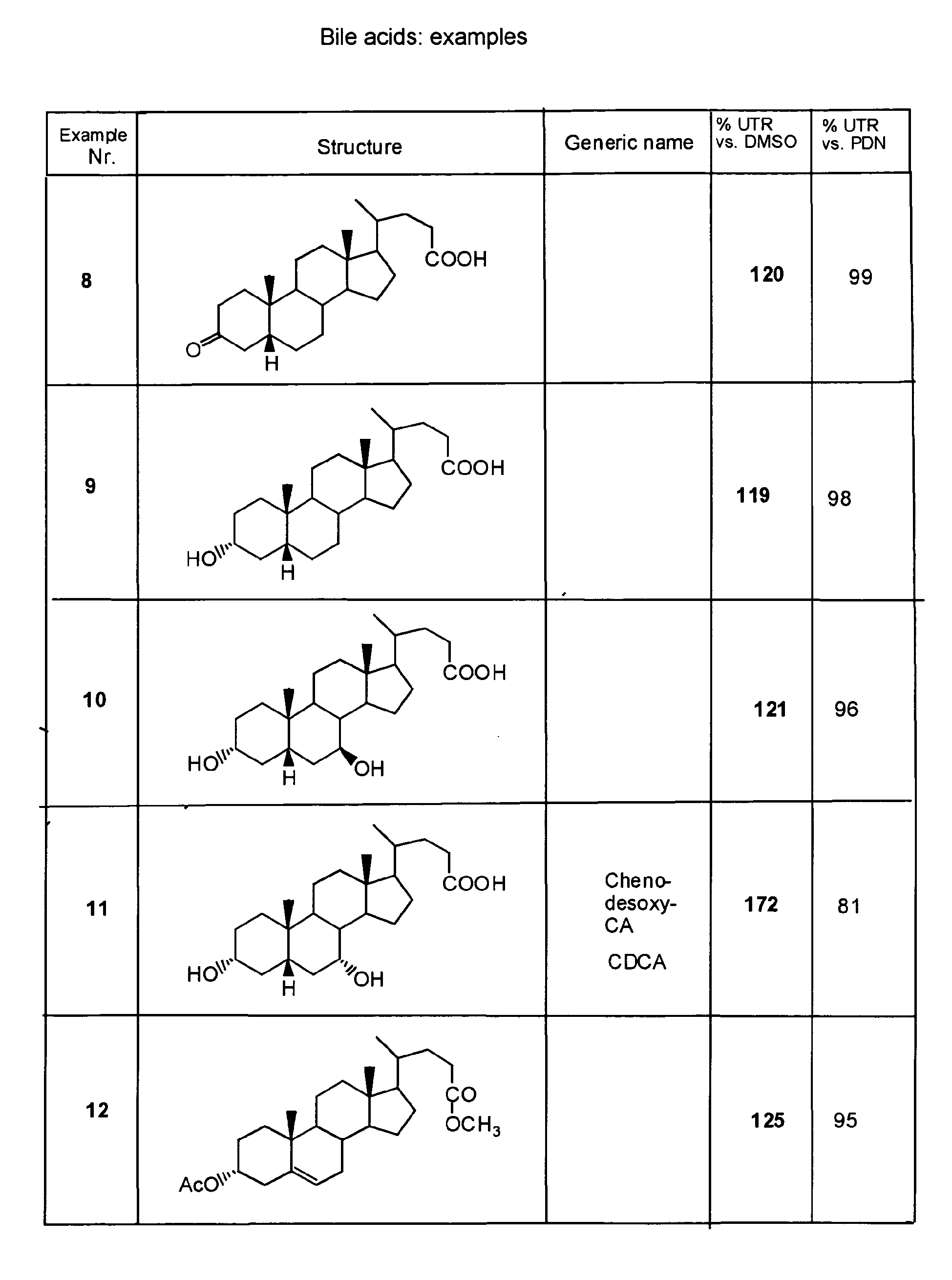

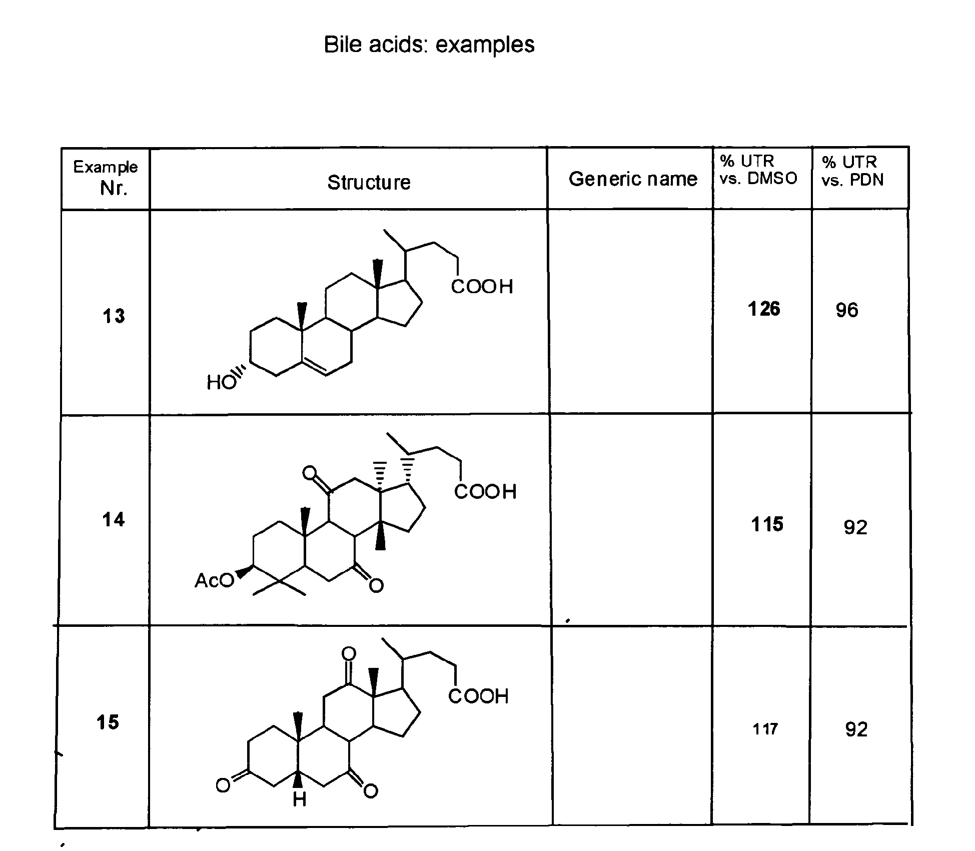

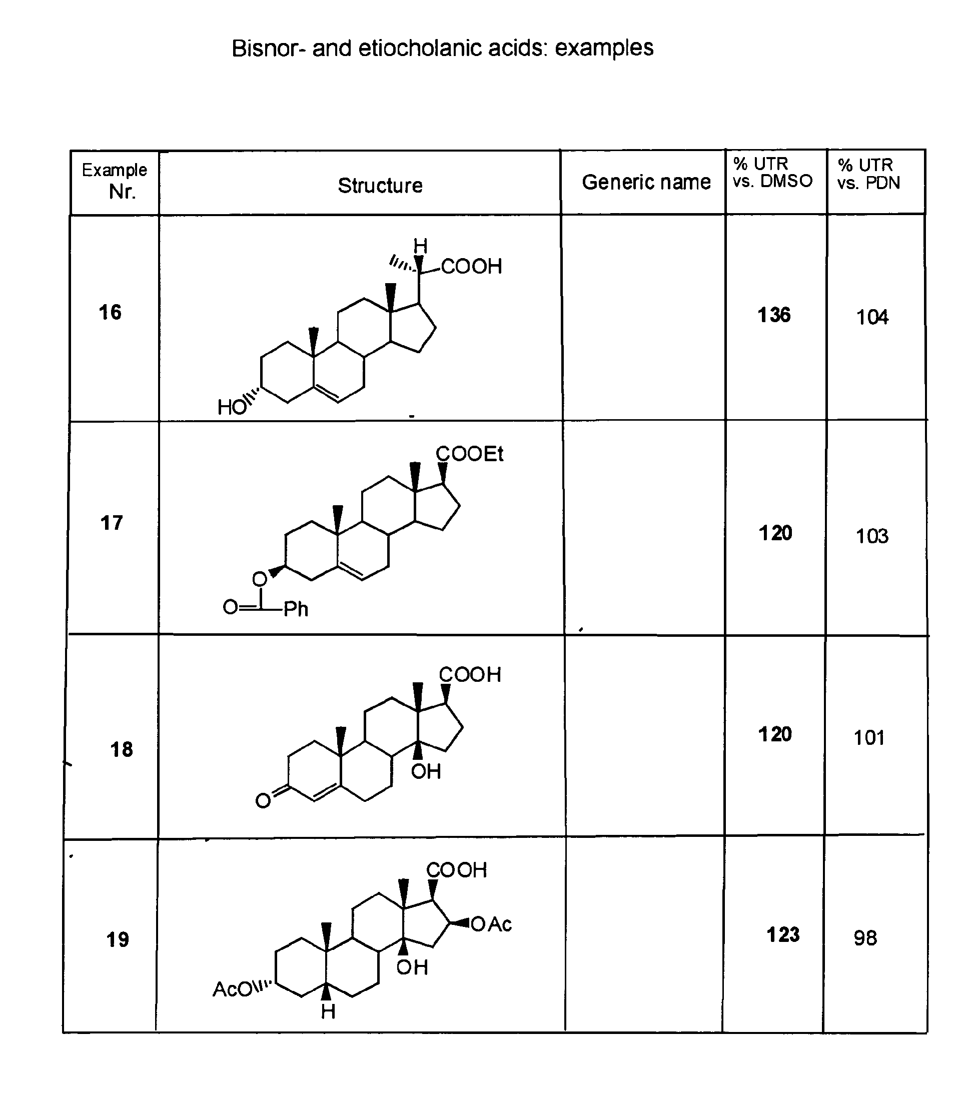

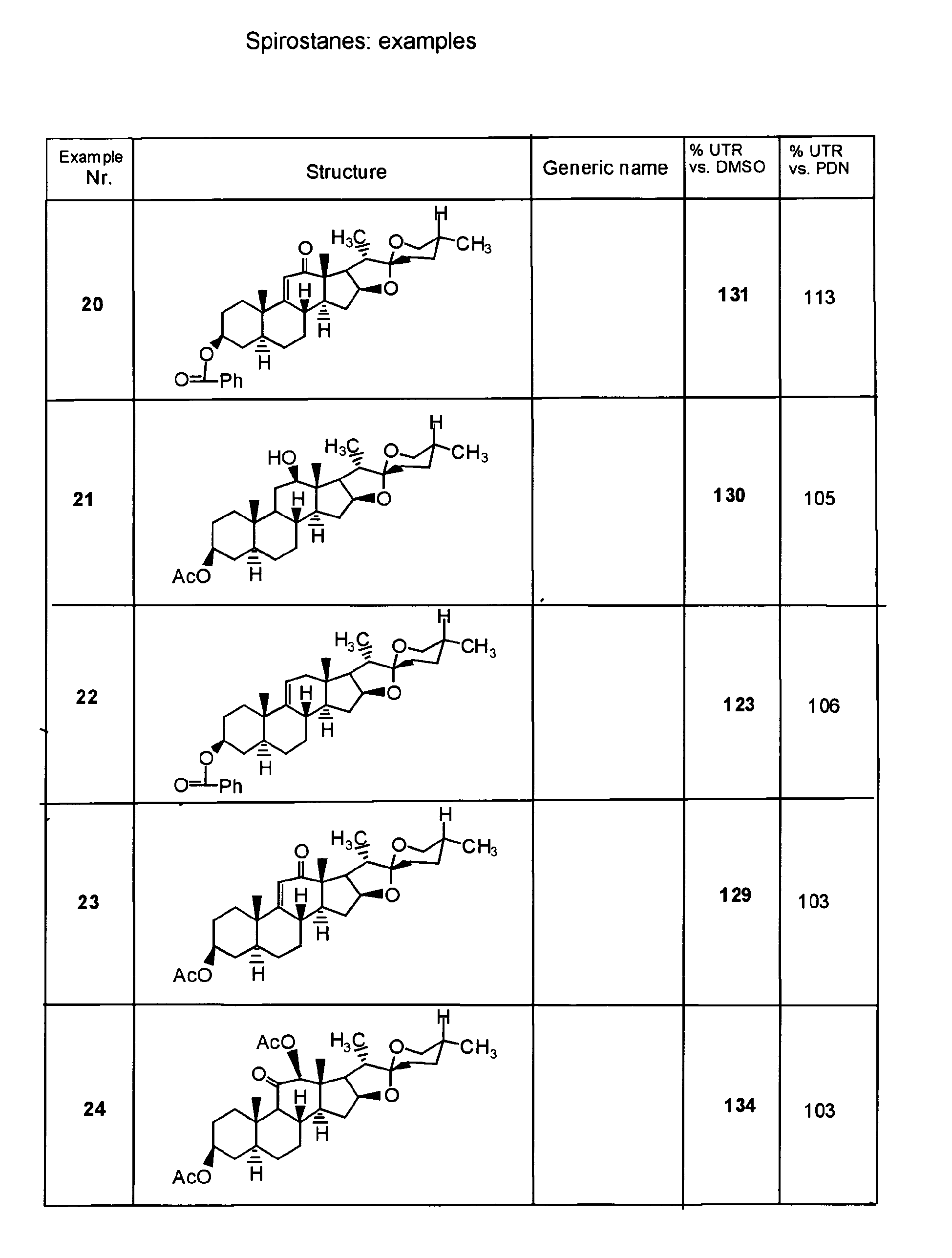

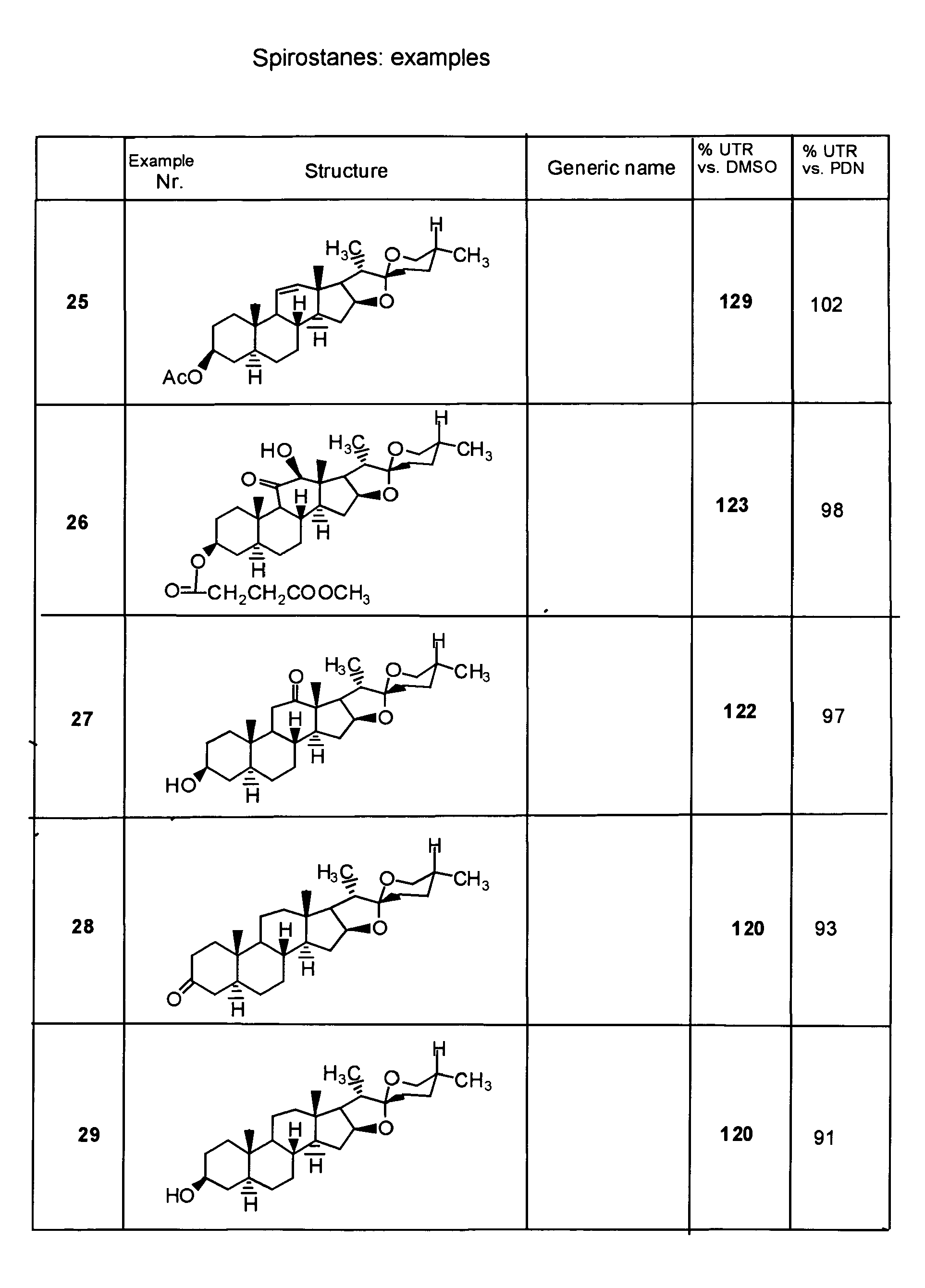

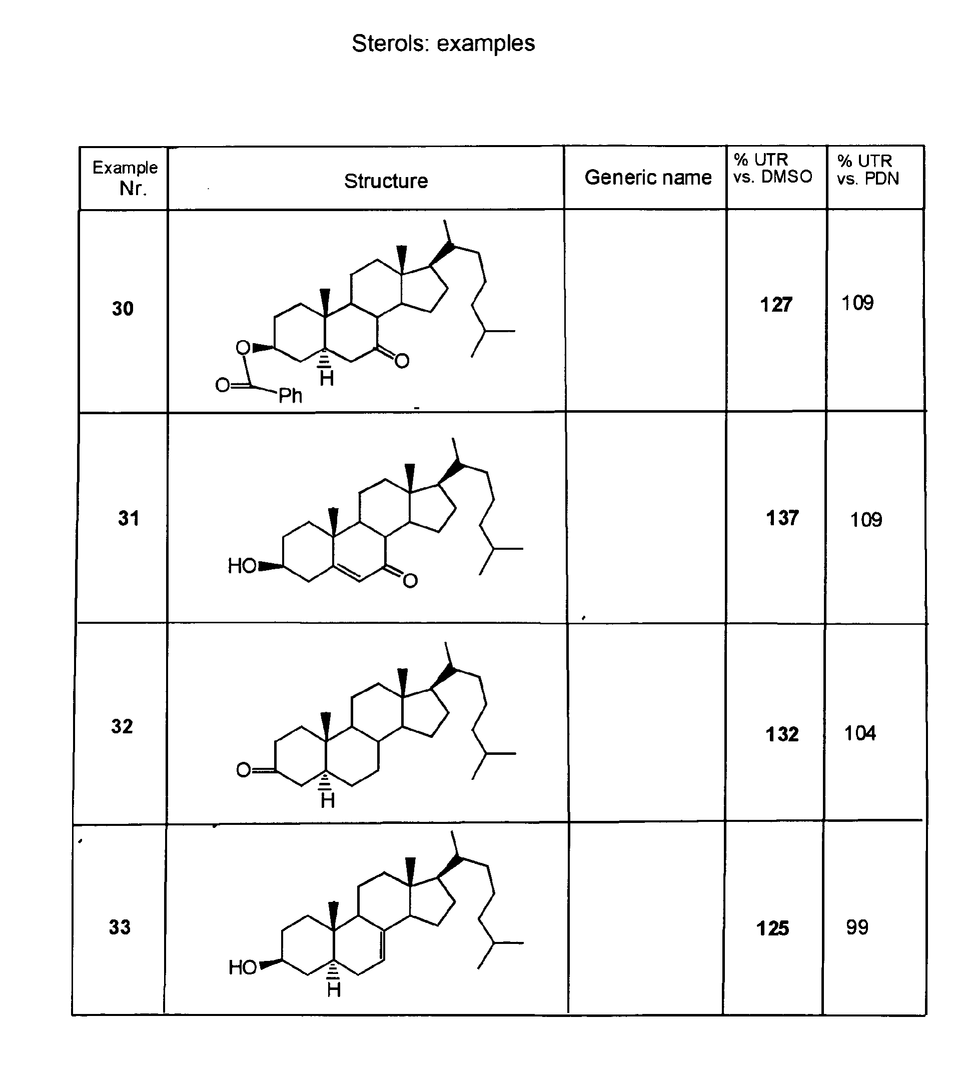

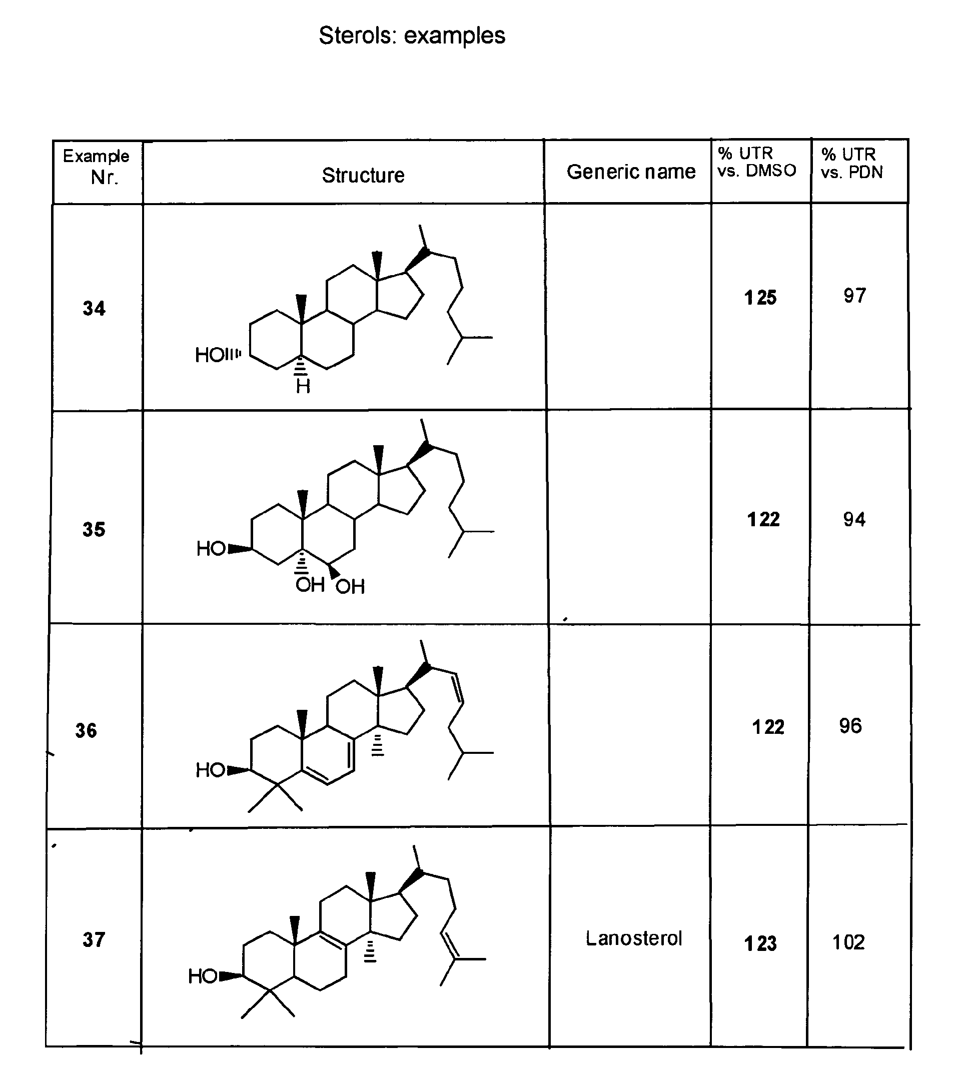

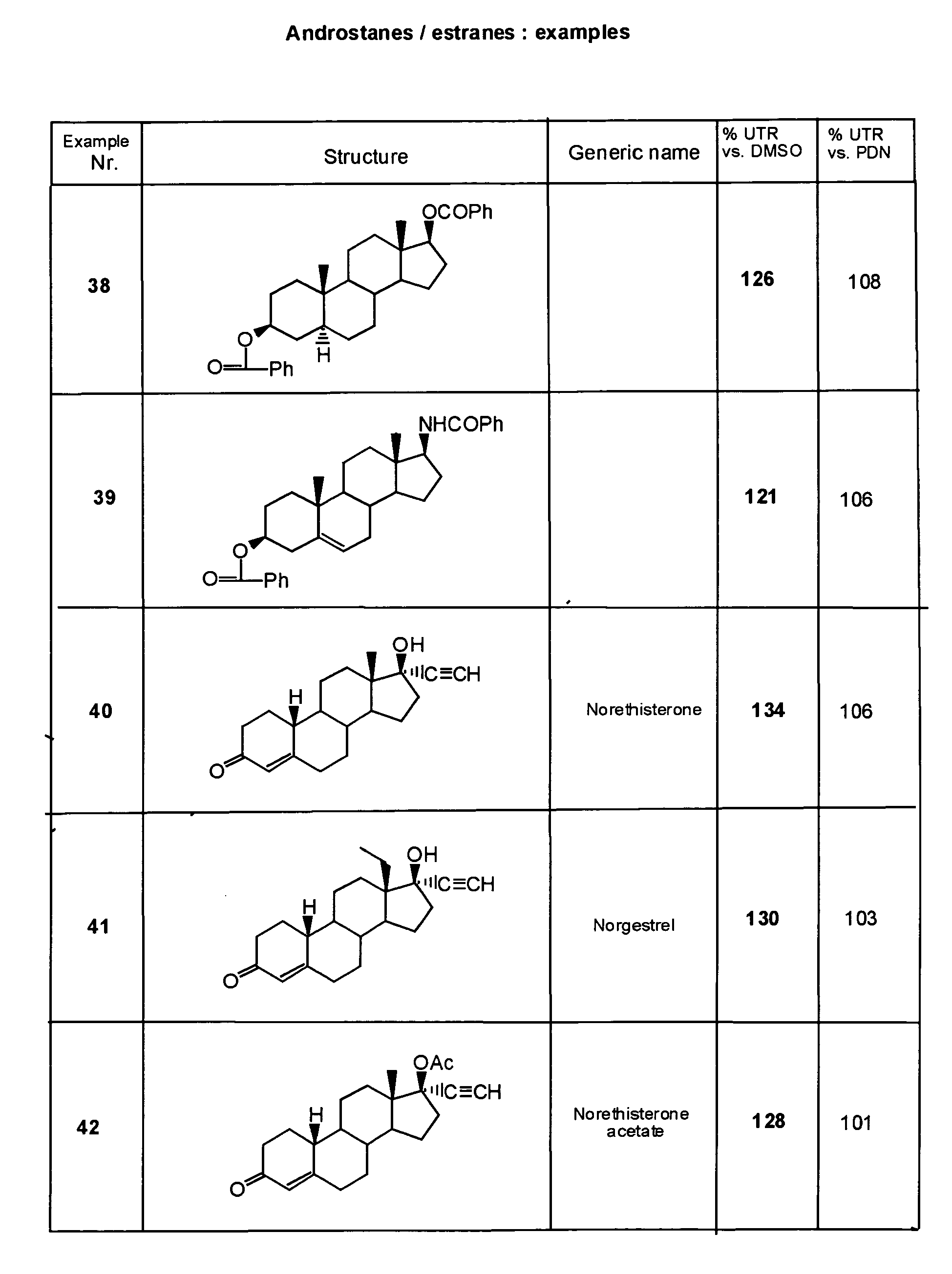

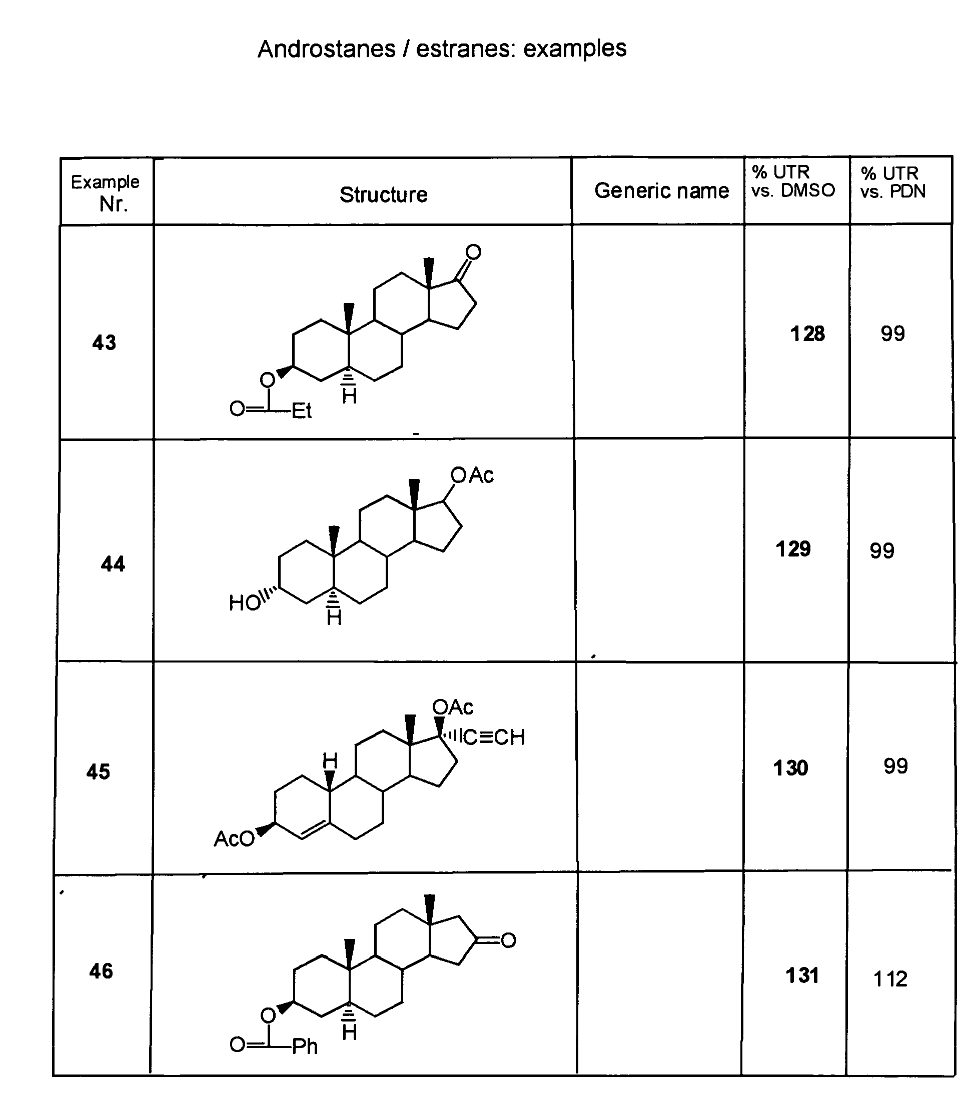

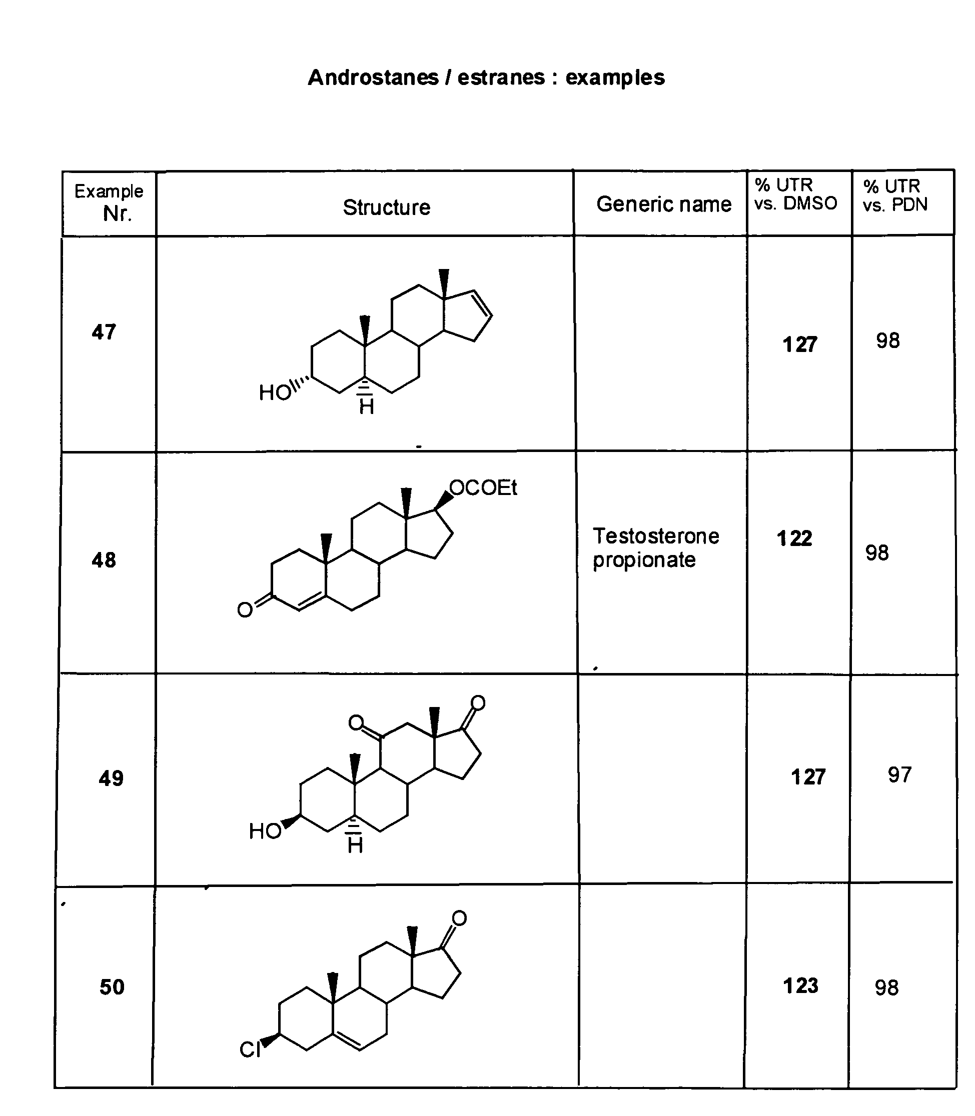

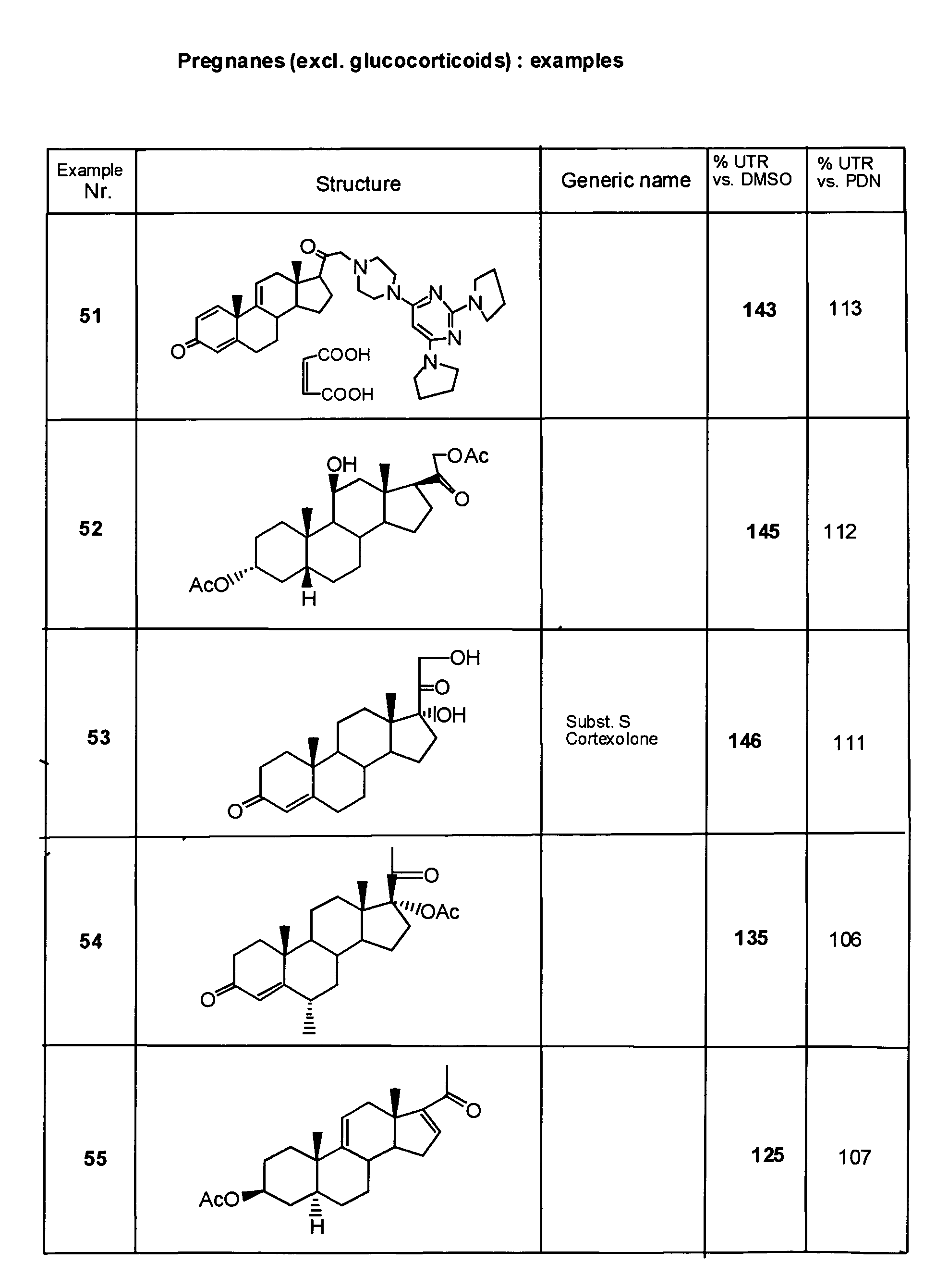

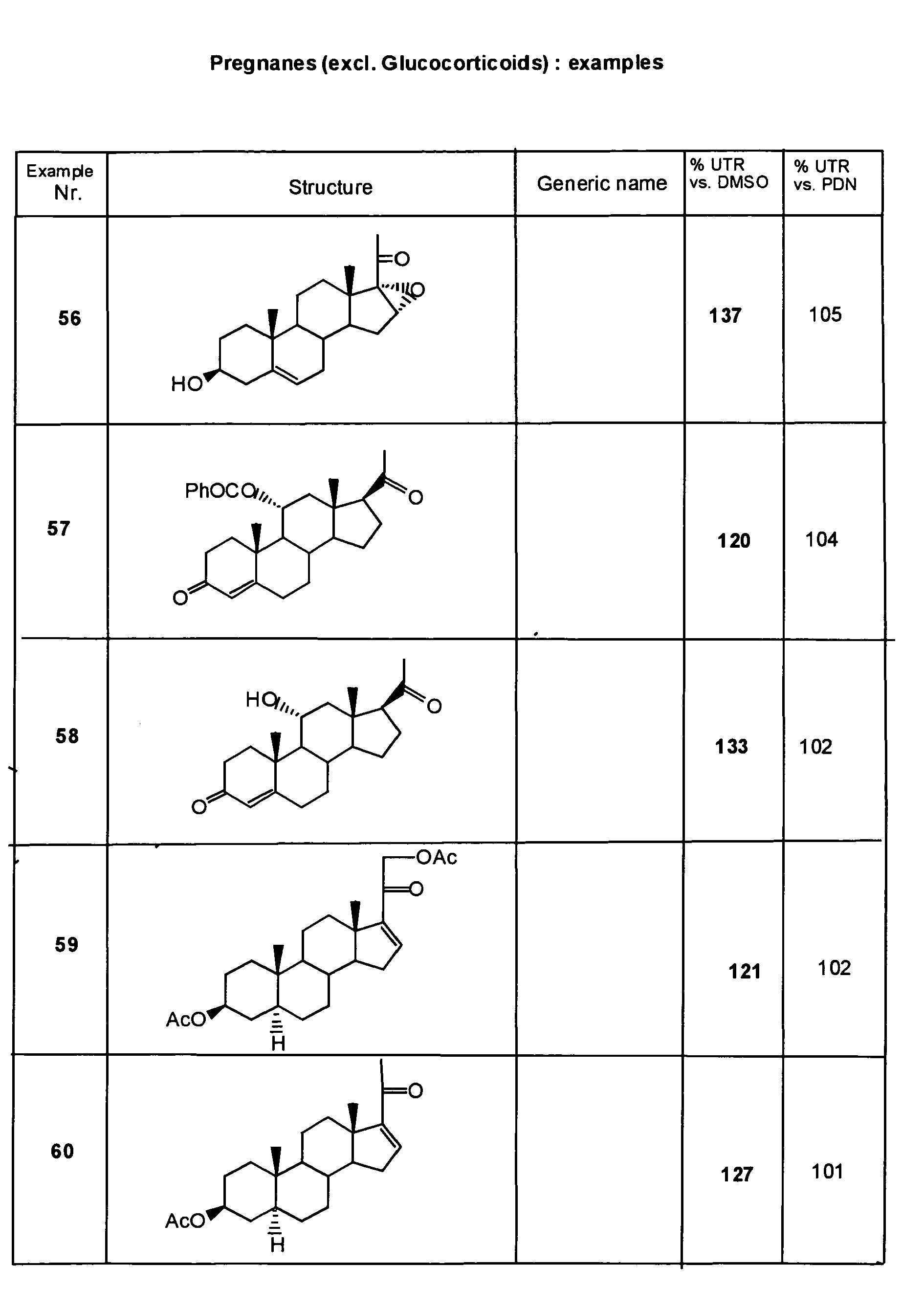

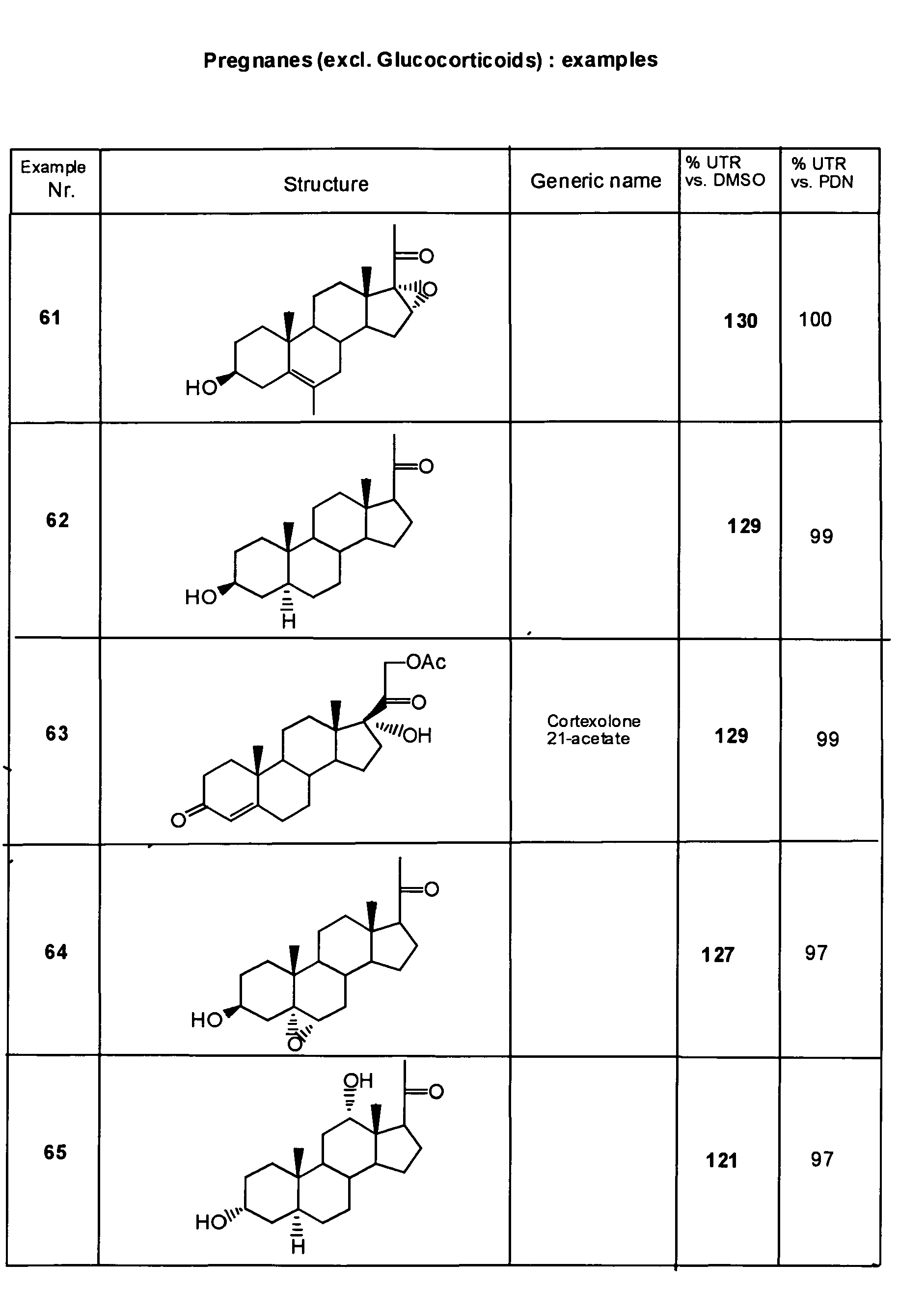

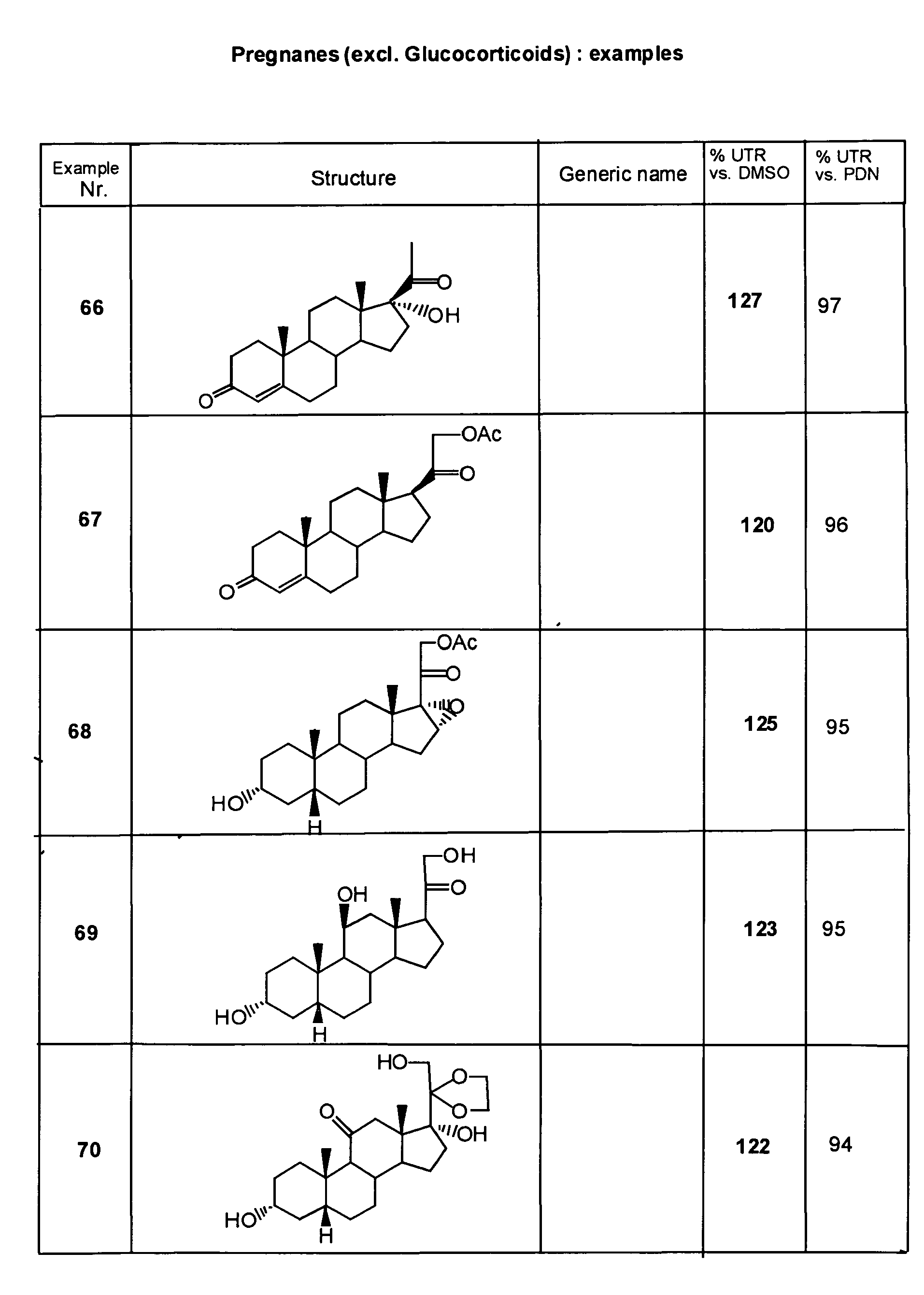

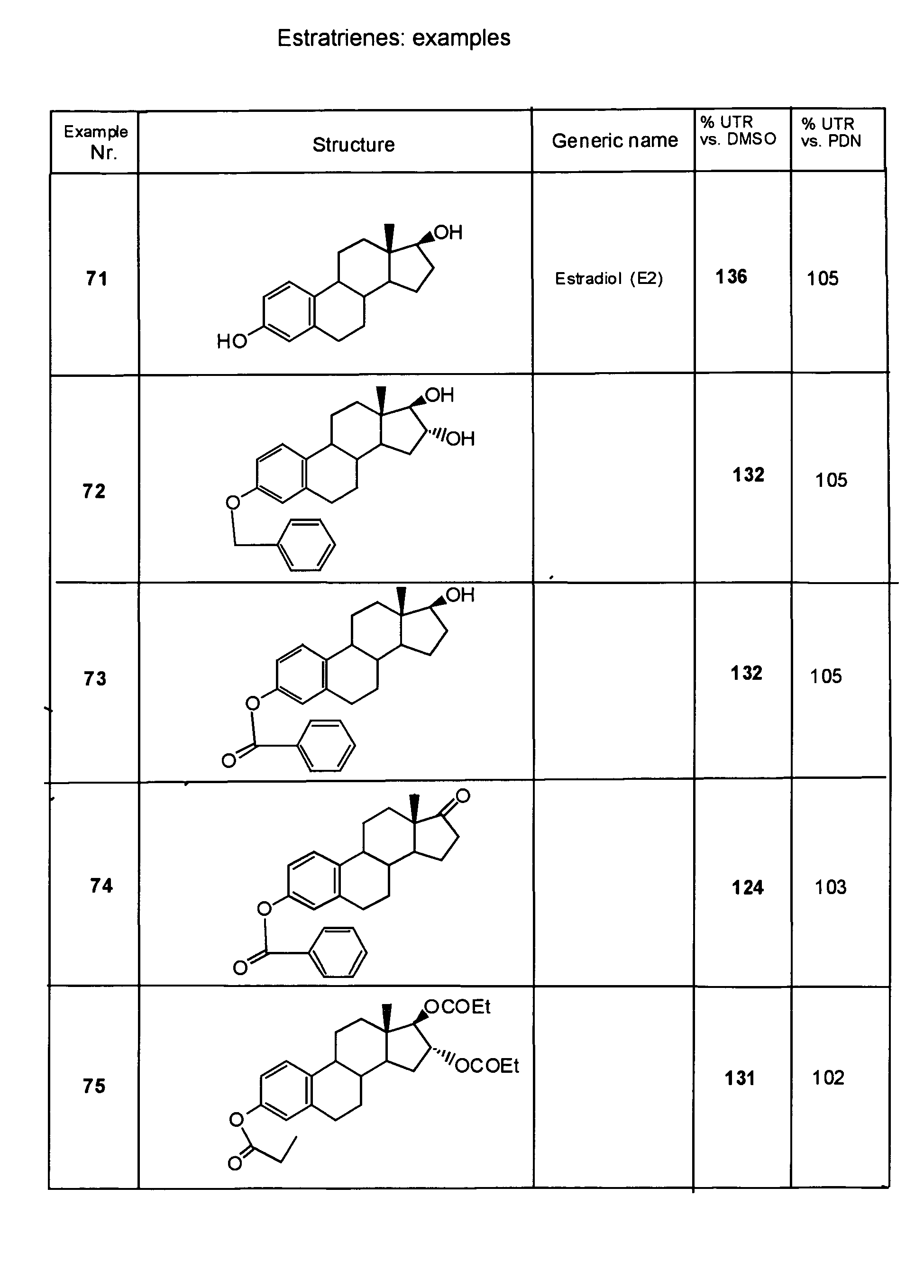

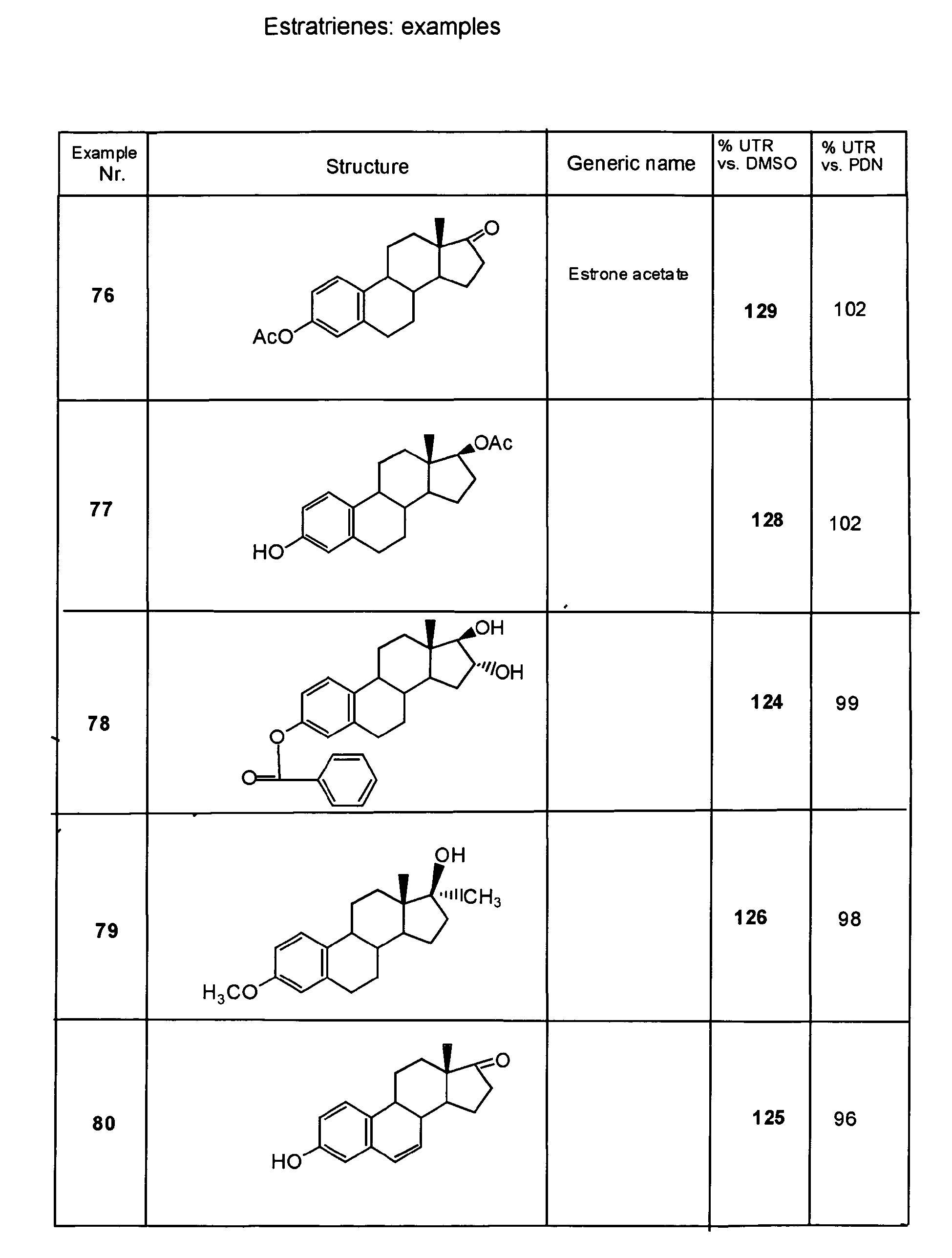

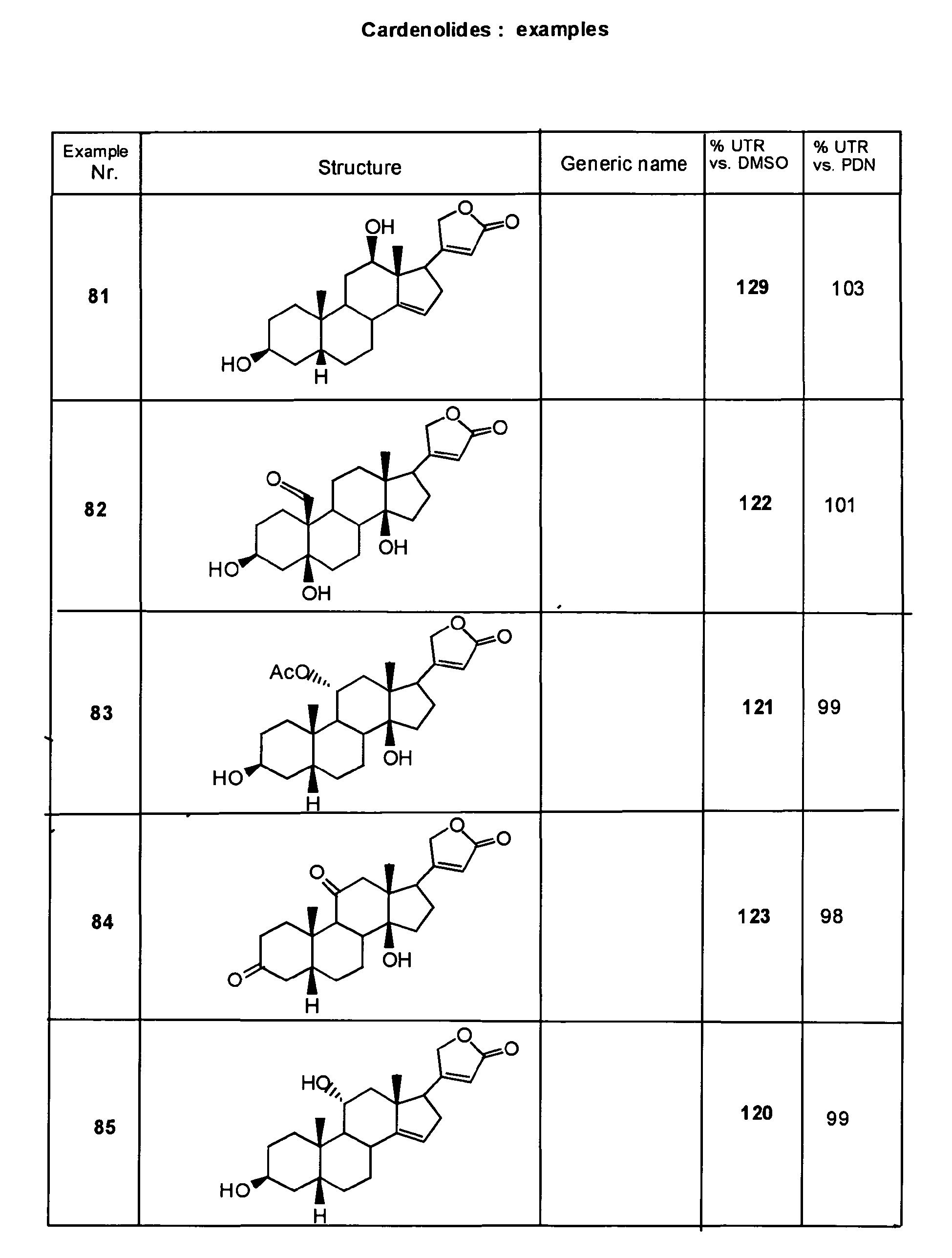

Surprisingly, it was found that several classes of non-glucocorticoid steroids were able to increase levels of utrophin protein to an extent comparable to the levels of utrophin protein induced by glucocorticoid steroids, such as 6α-methylprednisolone-21 sodium succinate (termed prednisolone or PDN). The steroid classes disclosed herein comprise compounds selected from bile acids (cholanic acids), bis-nor- and etiocholanic acids, spirostanes, sterols, androstanes and estranges, pregnanes, estratrienes, and cardenolides. The structural and pharmacological properties of these steroid compounds claimed in this invention are distinct from glucocorticoid-steroids and, consequently, these "non-glucocorticoid steroids" are not expected to cause the undesirable side effects in human patients typically observed with glucocorticoids. The combination of utrophin-inducing/increasing properties and the absence of glucocorticoid-specific side effects render these "non-glucocorticoid steroids" as a therapeutic means to ameliorate muscle wasting, in particular in DMD and BMD patients.

## Claims

1. Use of a compound selected form non-glucocorticoid steroids for the preparation of a medicament for the treatment of a disease susceptible to an increase of utrophin levels.

2. Use of a compound selected from non-glucocorticoid steroids for the preparation of a medicament for the treatment of a disease associated with loss of the dystrophin-DAPC complex.

3. Use according to claim 1 or 2, **characterized in that** the compound is capable of increasing utrophin expression and/or of increasing utrophin protein level in a cell.

4. Use according to any of claims 1 to 3, **characterized in that** the non-glucocorticoid steroid is a bile acid, a bis-nor- or etiocholanic acid, a spirostane, a sterol, an androstane, an estrane, a pregnane, an estratriene, or a cardenolide.

5. Use according to any of claims 1 to 4, **characterized in that** the compound has one of the following formulas: 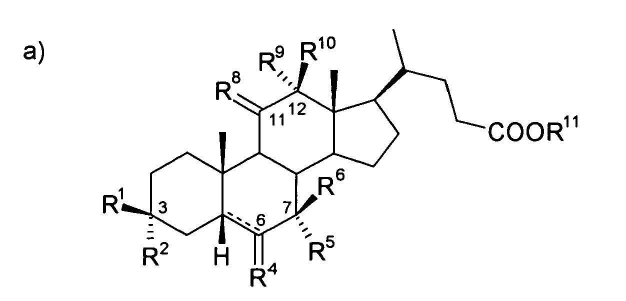 the substituents being as follows:
| | | |
|---|---|---|
| R¹ + R² = O | R⁵ + R⁶ = O | R⁵ = OR⁷; R⁶ = H |
| R¹ = OR³; R² = H | R⁵ + R⁶ = H₂ | R⁸ = O; H₂ |
| R¹ = H; R² = OR³ | R⁵ = H; R⁶ = OR⁷ | R⁹ + R¹⁰ = O; H₂ |
| R³ = H; acyl | R⁷ = H; acyl | R⁹ = OR⁷; R¹⁰ = H |
| R⁴ = H₂; O | | R¹¹ = H; Alkyl |

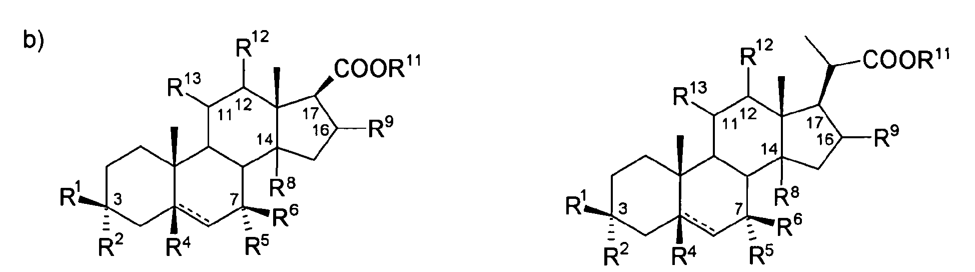
the substituents being as follows
| | | |
|---|---|---|
| R¹ + R² = O | R⁵ + R⁶ = O | R⁵ = OR⁷; R⁶ = H |
| R¹ = OR³; R² = H | R⁵ + R⁶ = H₂ | R⁸ = α- or β-H; β-OH |
| R¹ = H; R² = OR³ | R⁵ = H; R⁶ = OR⁷ | R⁹ = OR⁷ |
| R³ = H; acyl | R⁷ = H; acyl | |
| R⁴ = α- or β-H | | |
| | R¹¹ = H; Alkyl | |
| | R¹² = H; OR⁷ | |
| | R¹³ = H, OR⁷ | |

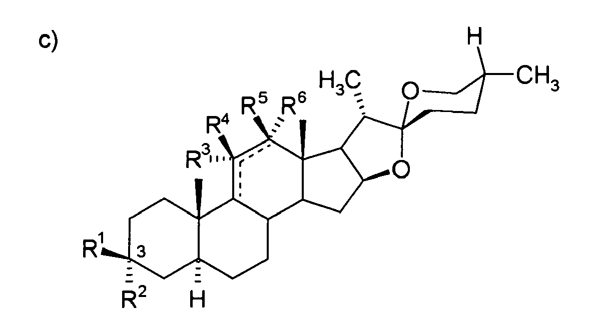
the substituents being as follows:
| | | |
|---|---|---|
| R¹ = OR⁷; R² = H | R³ = OR⁷; R⁴ = H | R⁵ = OR⁷; R⁶ = H |
| R¹ = H; R² = OR⁷ | R³ = H; R⁴ = OR⁷ | R⁵ = H; R⁶ = OR⁷ |
| R¹ + R² = O | R³ + R⁴ = O, H₂ | R⁵ + R⁶ = O, H₂ |
| | | |
| | R⁷ = H, Acyl | |

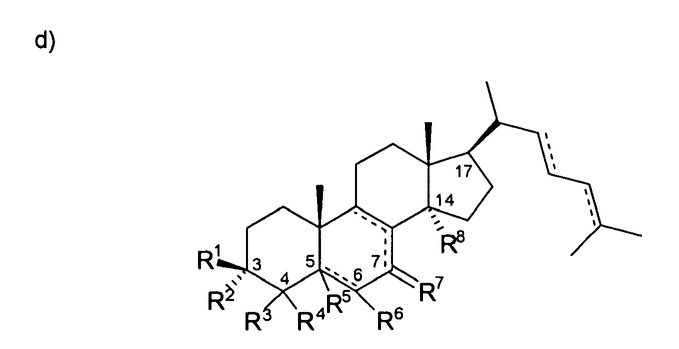
the substituents being as follows:
| | | |
|---|---|---|
| R¹ = OR⁹; R² = H | R³ = R⁴ = H; CH₃ | R⁷ = O; H₂ |
| R¹ = H; R² = OR⁹ | R⁵ = H; OH | R⁸ = H; CH₃ |
| R¹ + R² = O | R⁶ = H; OR⁹ | R⁹ = H; Acyl |

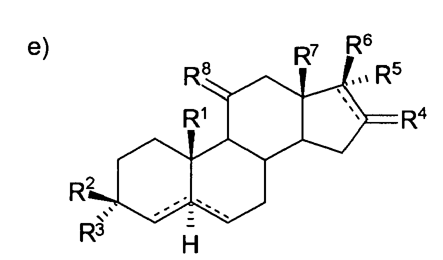
the substituents being as follows:
| | | |
|---|---|---|
| R¹ = H; CH₃ | R⁴ = H₂; O | R⁵ + R⁶ = O; H₂ |
| R² = OH; OAcyl; Hal | R⁵ = H; alkyl; alkenyl; alkynyl | R⁷ = CH₃; C₂H₅ |
| R³ = H | | R⁸ = H₂; O |
| R² + R³ = O | R⁶ = H; OH; OAcyl | |

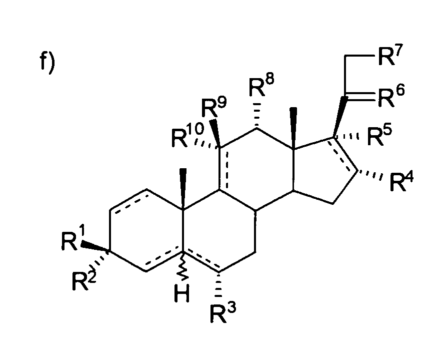
the substituents being as follows:
| | | |
|---|---|---|
| R¹ = OH; OAcyl | R⁴ = R⁵ = H | R⁸ = H; OH; OAcyl |
| R² = H | R⁴ + R⁶ = epoxy | R⁹ = H; OH |
| R¹ + R² = O | R⁶ = -O-CH₂-CH₂-O-; O | R¹⁰ = H; OH; OAcyl |
| R³ = H; CH₃ | R⁷ = H; OH; OAcyl | |

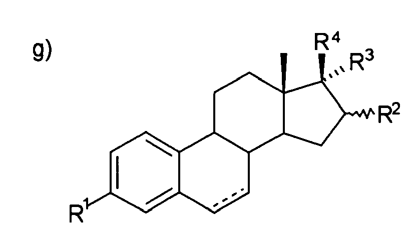
the substituents being as follows:
R¹ = OH, OAcyl
R² = OH; OAcyl; H
R³ = H; OH; OAcyl; alkyl
R⁴ = H; OH; OAcyl
R³ + R⁴ = O 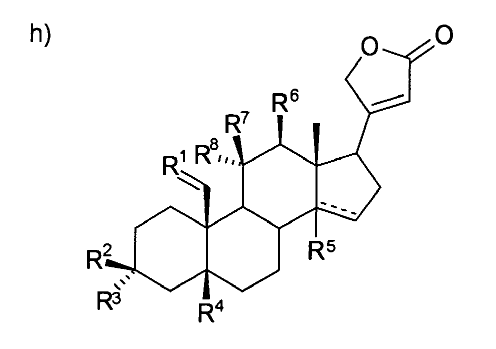
the substituents being as follows:
| | | |
|---|---|---|
| R¹ = H₂; O | R⁴ = H; OH | R⁷ = H; OH; OAcyl |
| R² = H; OR⁹ | R⁵ = H; OH | R⁸ = H; OH; OAcyl |
| R³ = H; OR⁹ | R⁶ = H; OH; OAcyl | R⁷ + R⁸ = O |
| R² + R³ = O | | R⁹ = H; Acyl |

6. Use according to any of claims 1 to 5, **characterized in that** the compound is capable of increasing the level of utrophin protein in human muscle cells.

7. Use according to any of claims 1 to 6, **characterized in that** the compound is capable of increasing the level of utrophin by at least 10%, preferably at least 20%, more preferably at least 50%, most preferably at least 100% over the solvent control.

8. Use according to any of claims 1 to 7, **characterized in that** a further active agent is used for simultaneous, separate or sequential use with the non- glucocorticoid steroid.

9. Use according to claim 8, **characterized in that** the further active agent is an agent suitable for treating Duchenne Muscular Dystrophy (DMD) or Becker Muscular Dystrophy (BMD).

10. Use according to any of claims 8 to 9, **characterized in that** the further active agent is selected from anti-oxidants, creatine and glucocorticoids.

11. Use according to any of claims 8 to 10, **characterized in that** the further active agent is selected from Vitamine E, CoQ10 and idebenone.

12. Use according to any of claims 1 to 11, **characterized in that** the expression and/or protein level of any of the dystrophin and DAPC protein members is reduced in the patient.

13. Use according to any of claims 1 to 11, **characterized in that** the disease is **characterized by** a reduction or loss of dystrophin.

14. Use according to any of claims 1 to 13, **characterized in that** the disease is a neuromuscular disease.

15. Use according to any of claims 1 to 14, **characterized in that** the disease is a muscle dystrophy or a related disorders such as muscular dystrophies, including dystrophinopathies and sarcoglycanopathies, limb girdle muscular dystrophies, congenital muscular dystrophies, congenital myopathies, distal and other myopathies and myotonic syndromes.

16. Use according to any of claims 1 to 15, **characterized in that** the disease is Duchenne Muscular Dystrophy or Becker Muscular Dystrophy.

17. Use according to any of claims 1 to 16 **characterized in that** the preparation is for oral administration.
